(19) Europäisches Patentamt
European Patent Office
Office européen des brevets

(11) Veröffentlichungsnummer: **0 023 594**
**B1**

(12) # EUROPÄISCHE PATENTSCHRIFT

(45) Veröffentlichungstag der Patentschrift: **16.05.84**

(51) Int. Cl.³: **C 07 D 239/90,** A 61 K 31/505

(21) Anmeldenummer: **80103896.9**

(22) Anmeldetag: **08.07.80**

(54) **Trans-Chinazolinderivate, Verfahren zu ihrer Herstellung, Zwischenprodukte und Arzneimittel enthaltend solche Chinazolinderivate.**

(30) Priorität: **09.07.79 US 55728**
**23.04.80 US 142903**

(43) Veröffentlichungstag der Anmeldung:
**11.02.81 Patentblatt 81/6**

(45) Bekanntmachung des Hinweises auf die Patenterteilung:
**16.05.84 Patentblatt 84/20**

(84) Benannte Vertragsstaaten:
**AT BE CH DE FR GB IT LI LU NL SE**

(56) Entgegenhaltungen:
**DE - A - 2 106 946**
**US - A - 2 786 055**
**US - A - 3 517 005**
**US - A - 3 696 102**

(73) Patentinhaber: **F. HOFFMANN-LA ROCHE & CO. Aktiengesellschaft, CH-4002 Basel (CH)**

(72) Erfinder: **Le Mahieu, Ronald Andrew, 18 Spruce Road, North Caldwell, N.J. (US)**
Erfinder: **Nason, William Chester, 64 North Glen Road, Mountain Lakes, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Trans-Chinazolinderivate, Verfahren zu ihrer Herstellung, Zwischenprodukte und Arzneimittel enthaltend solche Chinazolinderivate.

Die Erfindung betrifft trans-Chinazolinderivate der allgemeinen Formel I

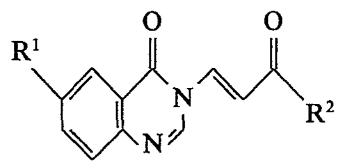

(I)

worin $R^1$ niederes Alkyl, niederes Cycloalkyl, niederes Alkoxy, Hydroxy, Halogen, niederes Alkylthio, niederes Alkylsulfinyl, niederes Alkylsulfonyl, di-($C_1$—$C_7$)Alkyl—$N(CH_2)_n$—O—, Hydroxy-niederes Alkoxy, Trifluormethyl, Nitro, Amino, mono-niederes Alkylamino oder di-niederes Alkylamino, $R^2$ Hydroxy, niederes Alkoxy, di-($C_1$—$C_7$)Alkyl—$N(CH_2)_n$—O— oder di-($C_1$—$C_7$)Alkyl—$N(CH_2)_n$—NH— und n eine ganze Zahl von 2—7 bedeuten,

und wenn $R^2$ Hydroxy bedeutet, Salze davon mit pharmazeutisch annehmbaren Basen, oder wenn $R^1$ Amino, mono-niederes Alkylamino, di-niederes Alkylamino oder di-($C_1$—$C_7$)Alkyl—$N(CH_2)_n$—O— und/oder $R^2$ di-($C_1$—$C_7$)Alkyl—$N(CH_2)_n$—O— oder di-($C_1$—$C_7$)Alkyl—$N(CH_2)_n$—NH— bedeuten, Salze davon mit pharmazeutisch annehmbaren Säuren.

Die neuen trans-3-(4-Oxo-4 H-chinazolin-3-yl)-2-propensäure-Derivate der allgemeinen Formel I sind wertvoll bei der Bekämpfung bzw. Verhütung von allergischen Reaktionen.

In der vorliegenden Beschreibung bedeutet der Ausdruck »niederes Alkyl« geradkettige oder verzweigte Alkylgruppen mit 1—7, vorzugsweise 1—4 Kohlenstoffatomen, beispielsweise Methyl, Äthyl, Propyl, Isopropyl, n-Butyl, t-Butyl, Neopentyl, n-Pentyl und n-Heptyl. Der Ausdruck »niederes Alkoxy« bedeutet eine Alkoxygruppe, in welcher die niedere Alkylgruppe wie oben definiert ist, beispielsweise Methoxy, Äthoxy, Propoxy und Pentoxy. Der Ausdruck »niederes Alkylthio« bedeutet eine Alkylthiogruppe, in welcher die niedere Alkylgruppe wie oben definiert ist, beispielsweise Methylthio, Äthylthio, Propylthio und Pentylthio. Der Ausdruck »niederes Alkylsulfinyl« bedeutet eine Alkylsulfinylgruppe, in welcher die niedere Alkylgruppe wie oben definiert ist, beispielsweise Methylsulfinyl und Äthylsulfinyl. Der Ausdruck »niederes Alkylsulfonyl« bedeutet eine Alkylsulfonylgruppe, in welcher die niedere Alkylgruppe wie oben definiert ist, beispielsweise Methylsulfonyl und Äthylsulfonyl.

Unter dem Ausdruck »niederes Cycloalkyl«, wie er in der vorliegenden Beschreibung verwendet wird, ist eine Cycloalkylgruppe mit 3—7 Kohlenstoffatomen, wie Cyclopropyl, Cyclopentyl und Cyclohexyl, zu verstehen. Der Ausdruck »Halogen« bedeutet Fluor, Chlor, Brom und Jod.

Der Ausdruck »Hydroxy-niederes Alkoxy« bedeutet eine niedere Alkoxygruppe, wie sie oben definiert wurde, in welcher ein Wasserstoffatom durch eine Hydroxygruppe ersetzt ist, beispielsweise 2-Hydroxyäthoxy oder 2-Hydroxypropoxy.

Der Ausdruck »mono-niederes Alkylamino« umfaßt Reste, wie Methylamino, Äthylamino und Isopropylamino. Der Ausdruck »di-niederes Alkylamino« umfaßt Reste, wie Dimethylamino, Diäthylamino, Methyl-äthylamino und Diisopropylamino.

Beispielhaft für »di-($C_1$—$C_7$)Alkyl—$N(CH_2)_n$—O—« sind Reste, wie Dimethylamino-äthoxy, Diäthylamino-äthoxy, Diisopropylamino-butoxy und Dipropylamino-äthoxy. Beispielhaft für »di-($C_1$—$C_7$)Alkyl—$N(CH_2)_n$—NH—« sind Reste, wie Dimethylamino-äthylamino, Diäthylamino-äthylamino, Äthylmethylamino-äthylamino und Diisopropylamino-äthylamino.

In einer bevorzugten Ausführungsform umfaßt die Erfindung Verbindungen der allgemeinen Formel I, worin $R^1$ niederes Alkyl, niederes Cycloalkyl, niederes Alkoxy oder niederes Alkylthio bedeutet. Besonders bevorzugte Verbindungen der allgemeinen Formel I sind solche, worin $R^1$ niederes Alkyl oder niederes Alkylthio und $R^2$ Hydroxy oder di-($C_1$—$C_7$)Alkyl-$N(CH_2)_n$—NH— bedeuten. Weitere besonders bevorzugte Verbindungen der Erfindung sind solche, worin $R^1$ Hydroxy oder niederes Alkoxy und $R^2$ Hydroxy bedeuten. Verbindungen der allgemeinen Formel I, worin $R^1$ niederes Alkylsulfinyl und $R^2$ Hydroxy bedeuten, sind ebenfalls bevorzugt. In einer weiteren bevorzugten Ausführungsform umfaßt die Erfindung Verbindungen der allgemeinen Formel I, worin $R^2$ Hydroxy bedeutet.

Ganz besonders bevorzugte Verbindungen der Erfindung sind:

trans-3-(6-Isopropyl-4-oxo-4 H-chinazolin-3-yl)-2-propensäure,
trans-3-(6-Methylthio-4-oxo-4 H-chinazolin-3-yl)-2-propensäure,
trans-3-(6-Isopropyl-4-oxo-4 H-chinazolin-3-yl)-2-propensäure-2-(diäthylamino)-äthylamid-hydrochlorid,
trans-3-(6-Isopropoxy-4-oxo-4 H-chinazolin-3-yl)-2-propensäure,
trans-3-(6-Hydroxy-4-oxo-4 H-chinazolin-3-yl)-2-propensäure und
trans-3-(6-Methylsulfinyl-4-oxo-4 H-chinazolin-3-yl)-2-propensäure.

Weitere bevorzugte Verbindungen der Erfindung sind:

trans-3-(6-Äthoxy-4-oxo-4 H-chinazolin-3-yl)-2-propensäure,
trans-3-(6-n-Propoxy-4-oxo-4 H-chinazolin-3-yl)-2-propensäure,
trans-3-(6-n-Butoxy-4-oxo-4 H-chinazolin-3-yl)-2-propensäure,
trans-3-(6-Methyl-4-oxo-4 H-chinazolin-3-yl)-2-propensäure,
trans-3-(6-Äthyl-4-oxo-4 H-chinazolin-3-yl)-2-propensäure,
trans-3-(6-n-Propyl-4-oxo-4 H-chinazolin-3-yl)-2-propensäure,
trans-3-(6-n-Butyl-4-oxo-4 H-chinazolin-3-yl)-2-propensäure,
trans-3-(6-Bromo-4-oxo-4 H-chinazolin-3-yl)-2-propensäure,
trans-3-(6-Fluoro-4-oxo-4 H-chinazolin-3-yl)-2-propensäure,
trans-3-(6-Nitro-4-oxo-4 H-chinazolin-3-yl)-2-propensäure,
trans-3-(6-Amino-4-oxo-4 H-chinazolin-3-yl)-2-propensäure,
trans-3-(6-Dimethylamino-4-oxo-4 H-chinazolin-3-yl)-2-propensäure,
trans-3-(6-Trifluoromethyl-4-oxo-4 H-chinazolin-3-yl)-2-propensäure,
trans-3-(6-t-Butyl-4-oxo-4 H-chinazolin-3-yl)-2-propensäure,
trans-3-(6-Isobutyl-4-oxo-4 H-chinazolin-3-yl)-2-propensäure und
trans-3-(6-sec-Butyl-4-oxo-4 H-chinazolin-3-yl)-2-propensäure.

Die Verbindungen der allgemeinen Formel I und ihre pharmazeutisch annehmbaren Salze mit Säuren und Basen werden erfindungsgemäß hergestellt, indem man

a) zur Herstellung von Verbindungen der allgemeinen Formel I a

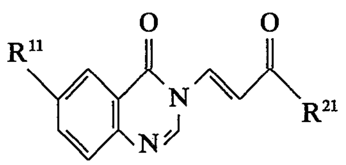

(I a)

worin $R^{11}$ niederes Alkyl, niederes Cycloalkyl, niederes Alkoxy, Nitro, Halogen, niederes Alkylthio, niederes Alkylsulfinyl, niederes Alkylsulfonyl, Trifluormethyl, di-niederes Alkylamino oder di-$(C_1-C_7)$Alkyl$-N(CH_2)_n-O-$, $R^{21}$ niederes Alkoxy, di-$(C_1-C_7)$Alkyl$-N(CH_2)_n-O-$ oder di-$(C_1-C_7)$Alkyl$-N(CH_2)_n-NH-$ und n eine ganze Zahl von 2—7 bedeuten, eine Verbindung der allgemeinen Formel II

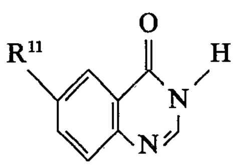

(II)

worin $R^{11}$ obige Bedeutung besitzt,
mit einem 3-Chloracrylsäure-Derivat der allgemeinen Formel III

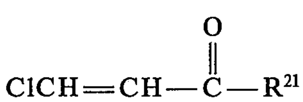

(III)

worin $R^{21}$ obige Bedeutung besitzt,
umsetzt, oder

b) zur Herstellung von Verbindungen der allgemeinen Formel I b

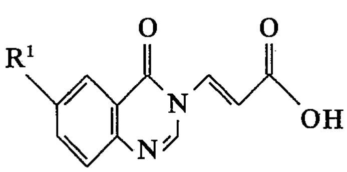

(Ib)

worin $R^1$ obige Bedeutung besitzt,
eine Verbindung der allgemeinen Formel I c

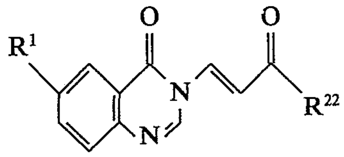

(Ic)

worin $R^{22}$ niederes Alkoxy bedeutet und $R^1$ obige Bedeutung besitzt, hydrolysiert, oder

c) zur Herstellung von Verbindungen der allgemeinen Formel I d

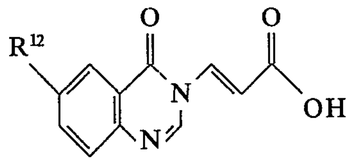

(Id)

worin $R^{12}$ niederes Alkylsulfinyl oder niederes Alkylsulfonyl bedeutet, eine Verbindung der allgemeinen Formel I e

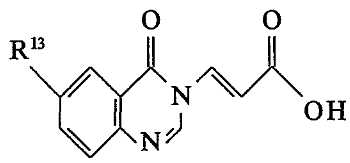

(Ie)

worin $R^{13}$ niederes Alkylthio bedeutet, oxydiert, oder

d) zur Herstellung von Verbindungen der allgemeinen Formel f

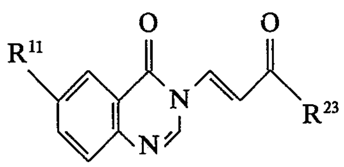

(If)

worin $R^{23}$ di-$(C_1{-}C_7)$Alkyl—$N(CH_2)_n$—O— oder di-$(C_1{-}C_7)$Alkyl—$N(CH_2)_n$—NH— bedeutet und $R^{11}$ und n obige Bedeutung besitzen, ein aktives Derivat einer Carbonsäure der allgemeinen Formel I g

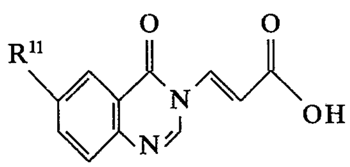

(Ig)

worin $R^{11}$ obige Bedeutung besitzt, mit einem Alkanol der allgemeinen Formel IV

$$\text{di-}(C_1{-}C_7)\text{Alkyl}{-}N(CH_2)_n{-}OH \qquad (IV)$$

oder mit einem Amin der allgemeinen Formel V

$$\text{di-}(C_1{-}C_7)\text{Alkyl}{-}N(CH_2)_n{-}NH_2 \qquad (V)$$

worin n jeweils obige Bedeutung besitzt, behandelt, oder

e) zur Herstellung von Verbindungen der allgemeinen Formel I h

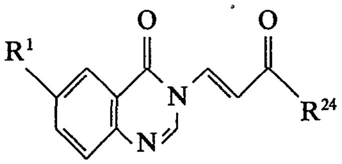

(Ih)

worin $R^{24}$ di-$(C_1{-}C_7)$Alkyl$-N(CH_2)_n-O-$ und $R^1$ und n obige Bedeutung besitzen, eine Carbonsäure der obigen allgemeinen Formel I b mit einem Halogenid der allgemeinen Formel VI

$$\text{di-}(C_1 - C_7)\text{Alkyl} - N(CH_2)_n - X \qquad \text{(VI)}$$

worin X Halogen bedeutet und n obige Bedeutung besitzt, behandelt, oder

f) zur Herstellung von Verbindungen der allgemeinen Formel I i

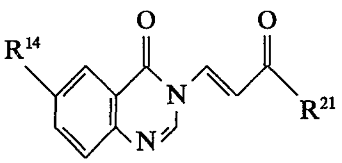

(I i)

worin $R^{14}$ Amino, mono-niederes Alkylamino, Hydroxy oder Hydroxy-niederes Alkoxy bedeutet und $R^{21}$ obige Bedeutung besitzt, in einer Verbindung der allgemeinen Formel VII

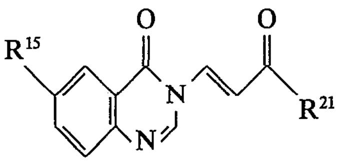

(VII)

worin $R^{15}$ eine geschützte Amino-, mono-niedere Alkylamino-, Hydroxy- oder Hydroxy-niedere Alkoxygruppe bedeutet und $R^{21}$ obige Bedeutung besitzt, die Schutzgruppe abspaltet, und

g) erwünschtenfalls eine Verbindung der allgemeinen Formel I, worin $R^2$ Hydroxy bedeutet, in ein pharmazeutisch annehmbares Salz mit einer Base überführt, oder eine Verbindung der allgemeinen Formel I, worin $R^1$ Amino, mono-niederes Alkylamino, di-niederes Alkylamino oder di-$(C_1{-}C_7)$Alkyl$-N(CH_2)_n-O-$ bedeutet und/oder $R^2$ di-$(C_1{-}C_7)$Alkyl$-N(CH_2)_n-O-$ oder di-$(C_1{-}C_7)$Alkyl$-N(CH_2)_n-NH-$ bedeutet und n obige Bedeutung besitzt, in ein pharmazeutisch annehmbares Säureadditionssalz überführt.

Gemäß Verfahrensvariante a) wird eine Verbindung der allgemeinen Formel I a hergestellt, indem man eine Verbindung der allgemeinen Formel II mit einem 3-Chloracrylsäure-Derivat der allgemeinen Formel III umsetzt.

Die N-3-Alkylierung einer Verbindung der allgemeinen Formel II mit einer Verbindung der allgemeinen Formel III, die in der trans-Konfiguration, d. h.

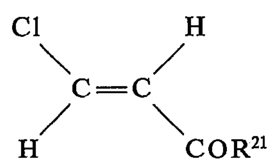

(III a)

oder in der cis-Konfiguration, d. h.

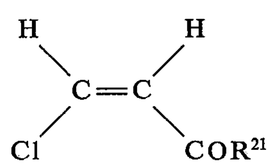

(III b)

worin $R^{21}$ jeweils obige Bedeutung besitzt, oder als Mischung davon vorliegen kann, kann in Gegenwart einer Base, beispielsweise einem Alkalimetallcarbonat, wie Kaliumcarbonat, einem Alkalimetallhydrid, wie Natriumhydrid, oder einem Alkalimetallalkoxyd, wie Natriummethoxyd, durchgeführt werden. Die N-3-Alkylierung kann in einem Temperaturbereich von etwa 25 bis etwa 100°C in einem Lösungsmittel, wie Dimethylformamid, Aceton oder Dimethylsulfoxyd, durchgeführt werden. Verwendet man Natriummethoxyd als Base, so kommen als Lösungsmittel auch niedere Alkanole, wie Methanol, in Frage. Vorzugsweise arbeitet man mit Kaliumcarbonat in Aceton bei Rückflußtemperatur. In einer weiteren bevorzugten Ausführungsform verwendet man Natriumhydrid in Dimethylformamid, und arbeitet in einem Temperaturbereich von etwa Raumtemperatur bis 100°C, wobei etwa 50°C bevorzugt werden.

Gemäß Verfahrensvariante b) wird eine Verbindung der allgemeinen Formel I b hergestellt, indem man eine Verbindung der allgemeinen Formel I c hydrolysiert. Diese Reaktion erfolgt beispielsweise mit einer Mineralsäure, wie Salzsäure, in einem Lösungsmittel, wie Wasser oder Essigsäure, vorzugsweise bei Rückflußtemperatur des gewählten Lösungsmittels.

Gemäß Verfahrensvariante c) wird eine Verbindung der allgemeinen Formel I d hergestellt, indem man eine Verbindung der allgemeinen Formel I e oxydiert. Insbesondere wird eine Verbindung der allgemeinen Formel I d, worin $R^{12}$ niederes Alkylsulfinyl bedeutet, erhalten, indem man eine Verbindung der allgemeinen Formel I e mittels bekannter Verfahren, beispielsweise mit einem Alkalimetallmetaperiodat, wie Natriummetaperiodat, vorzugsweise bei Raumtemperatur, oxydiert. Um eine Verbindung der allgemeinen Formel I d, worin $R^{12}$ niederes Alkylsulfonyl bedeutet, zu erhalten, wird eine Verbindung der allgemeinen Formel I e mittels bekannter Verfahren, beispielsweise mit Wasserstoffperoxid in Gegenwart einer niederen Fettsäure, wie Essigsäure, vorzugsweise in einem Temperaturbereich von 50 bis 100°C, oxydiert. Diese Oxydationen können aber auch nach anderen jedem Fachmann geläufigen Methoden, beispielsweise mit m-Chlorperbenzoesäure in einem halogenierten Kohlenwasserstoff, wie Methylenchlorid oder Chloroform, oder mit Peressigsäure, vorzugsweise in Essigsäure, durchgeführt werden.

Gemäß Verfahrensvariante d) wird eine Verbindung der allgemeinen Formel I f hergestellt, indem man ein aktives Derivat einer Carbonsäure der allgemeinen Formel I g mit einem Alkanol der allgemeinen Formel IV oder mit einem Amin der allgemeinen Formel V behandelt. Die freie Carbonsäure der allgemeinen Formel I g kann mit Reagenzien, wie Dicyclohexylcarbodiimid, N,N-Carbonyldiimidazol, Trifluoressigsäureanhydrid, p-Toluolsulfonsäurechlorid oder Mesitylensulfonsäurechlorid aktiviert werden. Ein bevorzugtes aktives Derivat ist das entsprechende Carbonsäurechlorid, das, wie jedem Fachmann bekannt, hergestellt werden kann, indem man die freie Carbonsäure der allgemeinen Formel I g z. B. mit überschüssigem Thionylchlorid behandelt. Das entsprechende Carbonsäurechlorid kann anschließend mit einer Verbindung der allgemeinen Formel IV (z. B. 2-Diäthylamino-äthanol) oder einer Verbindung der allgemeinen Formel V (z. B. 2-Diäthylamino-äthylamin oder 2-Diisopropylamino-äthylamin) in einem inerten organischen Lösungsmittel, beispielsweise in einem Kohlenwasserstoff, wie Benzol oder Toluol in einem Äther, wie Tetrahydrofuran oder Dimethoxyäthan oder in Dimethylformamid, in einem weiten Temperaturbereich, vorzugsweise bei Raumtemperatur, umgesetzt werden.

Gemäß Verfahrensvariante e) wird eine Verbindung der allgemeinen Formel I h hergestellt, indem man eine Carbonsäure der allgemeinen Formel I b mit einem Halogenid der allgemeinen Formel VI behandelt. Diese Reaktion kann durchgeführt werden, indem man eine Carbonsäure der allgemeinen Formel I b und ein Halogenid der allgemeinen Formel VI (z. B. 2-Diäthylamino-äthyl-chlorid) in einem inerten organischen Lösungsmittel, beispielsweise in einem Alkohol, wie Isopropylalkohol, vorzugsweise bei Rückflußtemperatur des gewählten Lösungsmittels, erhitzt.

Gemäß Verfahrensvariante f) wird eine Verbindung der allgemeinen Formel I i hergestellt, indem man in einer Verbindung der allgemeinen Formel VII die Schutzgruppe abspaltet. Bevorzugte Schutzgruppen sind solche, die unter milden Bedingungen entfernt werden können, beispielsweise Äther, wie Tetrahydropyranyläther oder Methoxyäthoxy-methyläther oder Silyläther, wie Trimethylsilyläther oder t-Butyldimethylsilyläther. Die für diese Reaktion benötigten Bedingungen, natürlich abhängig von der Art der verwendeten Schutzgruppe, können von jedem Fachmann leicht ermittelt werden.

Die oben erwähnten Schutzgruppen können beispielsweise mit einer Halogenwasserstoff-Säure, wie Salzsäure, in einem Temperaturbereich von etwa Raumtemperatur bis Rückflußtemperatur, vorzugsweise bei Rückflußtemperatur, entfernt werden.

Gemäß Verfahrensvariante g) bilden Verbindungen der allgemeinen Formel I, worin $R^2$ Hydroxy bedeutet, Salze mit pharmazeutisch annehmbaren Basen. Beispielhaft für solche Basen sind Alkalimetall-hydroxyde, wie Natriumhydroxid oder Kaliumhydroxyd, Erdalkali-Hydroxyde, wie Caleiumhydroxyd oder Bariumhydroxyd, Alkalimetall-alkoxyde, wie Natriumethanolat oder Kaliumethanolat, organische Basen, wie Piperidin, Diäthylamin, N-methylglucamin und N-(2-Aminoäthyl)-glycin.

Verbindung der allgemeinen Formel I, worin $R^1$ Amino, mono-niederes Alkylamino, di-niederes Alkylamino oder di-$(C_1{-}C_7)$Alkyl—$N(CH_2)_n$—O— bedeutet, und/oder $R^2$ di-$(C_1{-}C_7)$Alkyl—$N(CH_2)_n$—O— oder di-$(C_1{-}C_7)$Alkyl—$N(CH_2)_n$—NH bedeutet und n obige Bedeutung besitzt, bilden Salze mit pharmazeutisch annehmbaren Säuren. Beispielhaft für solche Säuren sind pharmazeutisch annehmbare organische und anorganische Säuren, wie Methansulfonsäure, p-Toluolsulfonsäure, Maleinsäure, Essigsäure, Salzsäure, Bromwasserstoffsäure und Schwefelsäure.

Solche Salze können im Hinblick auf den Stand der Technik und die Verbindungen von jedem Fachmann leicht hergestellt werden.

Ein als Ausgangsmaterial verwendetes Chinazolinon-Derivat der allgemeinen Formel II wird hergestellt, indem man ein Anthranilsäure-Derivat der allgemeinen Formel VIII

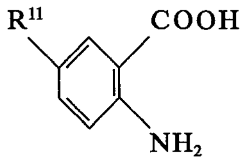

(VIII)

worin $R^{11}$ obige Bedeutung besitzt,
mit Formamid in einem Temperaturbereich von etwa 120 bis etwa 190°C, vorzugsweise ohne zusätzliches Lösungsmittel, behandelt.

Die Verbindungen der allgemeinen Formel VIII gehören einer bekannten Stoffklasse an und können nach bekannten Verfahren hergestellt werden.

Die 3-Chloracryl-Derivate der allgemeinen Formel III sind bekannt oder werden nach bekannten Methoden hergestellt. Beispielsweise werden die 3-Chloracryl-Derivate der allgemeinen Formel III c

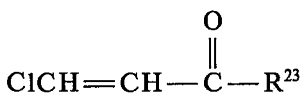

(III c)

worin $R^{23}$ obige Bedeutung besitzt,
hergestellt, indem man ein aktives Derivat der 3-Chloracrylsäure, beispielsweise das Anhydrid, ein gemischtes Anhydrid, das Imidazolid oder ein Halogenid, vorzugsweise das bekannte Säurechlorid der Formel Cl–CH=CH–COCl, das in der trans-Konfiguration, d. h.

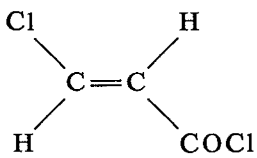

oder in der cis-Konfiguration, d. h.

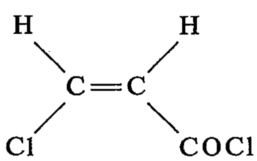

oder als Mischung davon vorliegen kann, mit einem Alkanol der allgemeinen Formel IV oder mit einem Amin der allgemeinen Formel V, in Analogie zur Herstellung von Verbindungen der allgemeinen Formel I f aus Verbindungen der allgemeinen Formel I g, gemäß Verfahrensvariante d), umsetzt.

Verbindungen der allgemeinen Formel VII werden hergestellt, indem man, in Analogie zu Verfahrensvariante a), ein geschütztes Chinazolinon-Derivat der allgemeinen Formel II'

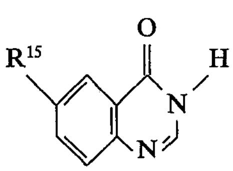

(II')

worin $R^{15}$ obige Bedeutung besitzt,
und ein 3-Chloracrylsäure-Derivat der allgemeinen Formel III als Ausgangsmaterialien verwendet.

Die Verbindungen der allgemeinen Formel VII sind neu und bilden ebenfalls Gegenstand der Erfindung.

Verbindungen der allgemeinen Formel II' werden hergestellt, indem man Anthranilsäure-Derivate der allgemeinen Formel VIII'

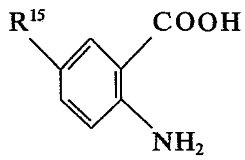

(VIII')

worin $R^{15}$ obige Bedeutung besitzt,

mit Formamid umsetzt, in Analogie zur Herstellung von Chinazolinon-Derivaten der allgemeinen Formel II aus Anthranilsäure-Derivaten der allgemeinen Formel VIII. Verbindungen der allgemeinen Formel VIII' sind bekannt oder werden nach bekannten Methoden hergestellt.

In einer weiteren erfindungsgemäßen Ausführungsform werden Chinazolinon-Derivate der allgemeinen Formel II' hergestellt, indem man in eine Verbindung der allgemeinen Formel II''

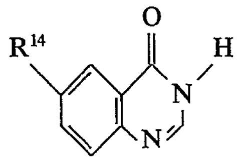

(II'')

worin $R^{14}$ obige Bedeutung besitzt,

eine Schutzgruppe einführt, beispielsweise mit einem Reagens, wie 3,4-Dihydro-2 H-pyran, Trimethylchlorsilan oder Methylvinyläther.

Die Verbindungen der allgemeinen Formel I und ihre pharmazeutisch annehmbaren Salze inhibieren Hautanaphylaxie bei Ratten und sind deswegen nützlich zur Verhinderung von allergischen Reaktionen, beispielsweise sind sie zur prophylaktischen Behandlung von Bronchialasthma brauchbar. Die antianaphylaktische Wirksamkeit kann durch den passiven Hautanaphylaxie-Test (PCA-Test) bei Ratten gezeigt werden. Bei diesem Test werden Ratten durch Injektion von Antisera durch die Haut passiv lokal sensibilisiert. Nach einer Latenzzeit von 24 Stunden wird die zu überprüfende Testverbindung, in diesem Fall ein erfindungsgemäßes trans-3-(4-Oxo-4 H-chinazolin-3-yl)-2-propensäure-Derivat, intraperitoneal verabreicht und 5 Minuten später Reagenz sowie Evans blue dye intravenös injiziert. die mit der lokalisierten Antigen-Antikörper-Reaktion einhergehenden Vorgänge im Körper führen zur Bildung von Hautschwellungen, deren Größen gemessen werden. Die Fähigkeit der Wirkstoffe, im Vergleich zu unbehandelten Tieren, bei den behandelten Tieren die Größe der entstandenen Hautschwellungen zu verkleinern, wird als Maß ihrer Wirksamkeit genommen.

Verwendet man erfindungsgemäße Verbindungen, wie z. B. trans-3-(6-Isopropyl-4-oxo-4 H-chinazolin-3-yl)-2-propensäure oder trans-3-(6-Methylthio-4-oxo-4 H-chinazolin-3-yl)-2-propensäure, als Testverbindungen in einer Dosis von 16 mg/kg intraperitoneal, so beträgt die Verminderung der Hautschwellung 100 bzw. 87 %.

Die anti-allergische Aktivität kann aber auch an der passiv sensibilisierten Ratte gezeigt werden (IgE). In diesem Test werden einer Ratte 18 Stunden vor der intravenösen Antigen (Eieralbumin)-Reizung Antisera verabreicht. Die Antigen-Reizung verursacht die IHR. Wenn eine anti-allergisch wirksame Verbindung intravenös vor der Antigen-Reizung verabreicht wird, hemmt sie die IHR und verhütet Bronchospasmus.

Wenn trans-3-(6-Methylthio-4-oxo-4 H-chinazolin-3-yl)-2-propensäure in obigem Test in einer oralen Dosis von 0,02 mg/kg verabreicht wird, beträgt die Hemmung von Bronchospasmus 50 %.

Die Verbindungen der allgemeinen Formel I und ihre pharmazeutisch annehmbaren Salze können oral oder parenteral als anti-allergische Mittel, z. B. für prophylaktische Behandlung von Bronchialasthma, in den individuellen Erfordernissen angepaßten Dosen verabreicht werden. Sie können therapeutisch, z. B. oral oder parenteral, nach Verarbeiten einer therapeutischen Dosis in eine übliche galenische Darreichungsform, wie Tabletten, Kapseln, Elixiere, Suspensionen oder Lösungen, verabreicht werden. Sie können in Mischungen mit üblichen pharmazeutischen Trägern oder Bindemitteln, wie beispielsweise Maisstärke, Calciumstearat, Magnesiumcarbonat, Calciumsilicat, Dicalciumphosphat, Kalk oder Lactose, verabreicht werden. Darüber hinaus können sie in Gegenwart von Puffer oder zur Einstellung der Isotonie verwendeten Mitteln verabreicht werden, und die pharmazeutischen Dosisformen können, wenn erwünscht, den üblichen pharmazeutischen Maßnahmen, wie z. B. Sterilisation, unterworfen werden. Wie oben angeführt, kann die Dosis den individuellen Erfordernissen angepaßt werden. Sie können auch andere therapeutisch wertvolle Substanzen enthalten.

Die Menge an Wirkstoff, welche in irgendeiner der oben beschriebenen Dosisformen vorhanden ist, ist variabel. Kapseln oder Tabletten enthaltend etwa 1 mg bis etwa 25 mg einer Verbindung der allgemeinen Formel I oder eine äquivalente Menge eines pharmazeutisch annehmbaren Salzes davon werden jedoch bevorzugt.

Die folgenden Beispiele sollen die Erfindung weiter erläutern, sie jedoch in keiner Weise einschränken. Alle Temperaturen sind in °C angegeben.

Beispiel 1

1,284 g Natriumhydrid (57%ige Öldispersion) werden mit Pentan gewaschen und in 25 ml wasserfreiem Dimethylformamid suspendiert. Unter einer Argon-Atmosphäre gießt man 4,0 g 6-Methoxy-4(3 H)-chinazolinon hinzu und rührt die Mischung während 10 Minuten bei Raumtemperatur und während 30 Minuten bei 50°. Nach Abkühlen auf 30° gibt man tropfenweise 3,02 g Methyl-trans-3-chloracrylat in 20 ml wasserfreiem Dimethylformamid hinzu. Anschließend wird der Ansatz während einer Stunde auf 50° erwärmt, in einem Eisbad abgekühlt und mit 100 ml Wasser versetzt. Der ausgefallene Festkörper wird abfiltriert und aus Methylenchlorid/Methanol umkristallisiert. Man erhält 4,30 g reinen trans-3-(6-Methoxy-4-oxo-4 H-chinazolin-3-yl)-2-propensäure-methylester vom Schmelzpunkt 184—186°.

Beispiel 2

Eine Mischung aus 4,30 g trans-3-(6-Methoxy-4-oxo-4 H-chinazolin-3-yl)-2-propensäure-methylester und 110 ml 6 N Salzsäure wird unter Rühren während 15 Minuten unter Rückfluß zum Sieden erhitzt. Nach Zugabe von 100 ml Wasser wird die Reaktionsmischung in einem Eisbad abgekühlt und vom ausgefallenen Material filtriert. Man erhält 3,13 g reine trans-3-(6-Methoxy-4-oxo-4 H-chinazolin-3-yl)-2-propensäure vom Schmelzpunkt 298—299°.

Beispiel 3

Eine Suspension von 0,862 g trans-3-(6-Methoxy-4-oxo-4 H-chinazolin-3-yl)-2-propensäure in 15 ml Wasser wird unter Rühren mit 3,5 ml 1,0 N Natriumhydroxid versetzt. Nach einigen Minuten wird die Mischung filtriert. Zum Filtrat gibt man 100 ml Pyridin und erhitzt die Mischung so lange zum sieden, bis der größte Teil des Wassers entfernt ist, und läßt abkühlen. Nach Abfiltrieren des auskristallisierten Materials erhält man 0,68 g reines trans-3-(6-Methoxy-4-oxo-4 H-chinazolin-3-yl)-2-propensäure-natriumsalz-hemihydrat vom Schmelzpunkt 329—330°.

Beispiel 4

Zu einer Lösung von 0,513 g trans-3-(6-Methoxy-4-oxo-4 H-chinazolin-3-yl)-2-propensäure in 35 ml Pyridin gibt man 0,248 g N-(2-Aminoäthyl)-glycin in 5 ml Wasser. Nach Einengen der Lösung im Vakuum wird der Rückstand in 150 ml Wasser und 3 ml Pyridin aufgenommen. Durch Lyophilisieren der Lösung erhält man 3-(6-Methoxy-4-oxo-4 H-chinazolin-3-yl)-2-propensäure-N-(2-aminoäthyl)-glycinsalz.

Beispiel 5

Eine Suspension von 1,65 g trans-3-(6-Methoxy-4-oxo-4 H-chinazolin-3-yl)-2-propensäure in 100 ml Isopropylalkohol wird unter Rühren zum Rückfluß erhitzt und tropfenweise mit 2,7 g 2-(Diäthylamino)-äthylchlorid in 30 ml Isopropylalkohol versetzt. Man erhitzt die Reaktionsmischung während 8 Stunden unter Rückfluß zum Sieden und läßt anschließend abkühlen. Der Niederschlag wird abfiltriert, mit 125 ml gesättigter Natriumbicarbonatlösung behandelt und mit Chloroform extrahiert. Durch Trocknen des Extraktes über Magnesiumsulfat und Eindampfen im Vakuum erhält man 2,3 g eines Festkörpers. Dieser Festkörper wird in 35 ml Methylenchlorid gelöst und mit 2,52 ml 2,65 N Salzsäure in Methanol versetzt. Anschließend gibt man soviel Äther dazu, daß die Lösung gerade trübe ist, und läßt über Nacht in der Kälte stehen. Durch Abfiltrieren erhält man 1,30 g reines trans-3-(6-Methoxy-4-oxo-4 H-chinazolin-3-yl)-2-propensäure-(2-diäthylaminoäthyl)-esterhydrochlorid vom Schmelzpunkt 217—218°.

Beispiel 6

Eine Mischung aus 5,38 g 5-Isopropylanthranilsäure und 4,82 ml Formamid wird unter Rühren während 4 Stunden auf 140—145° erhitzt und anschließend auf Raumtemperatur abgekühlt. Nach Entfernen von überschüssigem Formamid im Vakuum versetzt man mit Wasser, filtriert den Niederschlag ab und kristallisiert ihn aus Methylenchlorid/Hexan um. Man erhält 4,50 g reines 6-Isopropyl-4(3 H)-chinazolinon vom Schmelzpunkt 147°.

Beispiel 7

1,15 g Natriumhydrid (57%ige Öldispersion) werden mit Pentan gewaschen und in 25 ml wasserfreiem Formamid suspendiert. Unter einer Argon-Atmosphäre gibt man 3,86 g 6-Isopropyl-4(3 H)-chinazolinon hinzu und rührt die Mischung 30 Minuten bei Raumtemperatur und eine Stunde bei 50°. Nach Abkühlen auf 30° gibt man tropfenweise 2,68 g Methyl-trans-3-chloracrylat in 10 ml wasserfreiem Dimethylformamid hinzu und erhitzt die Reaktionsmischung während 1½ Stunden auf 50°. Nach Entfernen des Dimethylformamids im Vakuum versetzt man mit Wasser und filtriert vom ausgefallenen Festkörper ab. Durch Umkristallisieren aus Methylenchlorid/Methanol erhält man 2,73 g reinen trans-3-(6-Isopropyl-4-oxo-4 H-chinazolin-3-yl)-2-propensäure-methylester vom Schmelzpunkt 145°.

Beispiel 8

Eine Mischung aus 2,995 g trans-3-(6-Isopropyl-4-oxo-4 H-chinazolin-3-yl)-2-propensäure-methylester und 175 ml 6 N Salzsäure wird unter Rühren 20 Minuten zum Rückfluß erhitzt. Nach Abkühlen auf Raumtemperatur gibt man 175 ml Wasser hinzu und läßt die Reaktionsmischung über Nacht in der Kälte stehen. Durch Filtrieren erhält man 1,58 g reine trans-3-(6-Isopropyl-4-oxo-4 H-chinazolin-3-(6-isopropyl-4-oxo-4 H-chinazolin-3-yl)-2-propensäure vom Schmelzpunkt 245°.

Beispiel 9

Eine suspension von 1,29 g trans-3-(6-Isopropyl-4-oxo-4 H-chinazolin-3-yl)-2-propensäure in 20 ml Wasser wird unter Rühren mit 5,0 ml 1,0 N Natriumhydroxid versetzt. Nach einigen Minuten wird die Mischung filtriert. Das Filtrat wird mit 50 ml Wasser versetzt und lyophilisiert, dabei erhält man 1,40 g trans-3-(6-Isopropyl-4-oxo-4 H-chinazolin-3-yl)-2-propensäure-Natriumsalz, das 0,25 Mol Kristallwasser enthält.

Beispiel 10

Eine Lösung von 0,774 g trans-3-(6-Isopropyl-4-oxo-4 H-chinazolin-3-yl)-2-propensäure in 50 ml Pyridin wird mit 0,354 g N-(2-Aminoäthyl)-glycin in 5 ml Wasser versetzt. Nach Einengen der Lösung im Vakuum wird der Rückstand in 50 ml Wasser, das einige Tropfen Pyridin enthält, aufgenommen. Diese Lösung wird lyophilisiert und ergibt trans-3-(6-Isopropyl-4-oxo-4 H-chinazolin-3-yl)-2-propensäure-N-(2-aminoäthyl)-glycinsalz.

Beispiel 11

Eine Mischung aus 1,77 g 5-Cyclopropyl-anthranilsäure und 1,6 ml Formamid wird unter Rühren während 4 Stunden auf 140—145° erwärmt. Nach Abkühlen auf Raumtemperatur wird überschüssiges Formamid im Vakuum entfernt. Nach Zugabe von Wasser wird der Niederschlag abfiltriert und aus Methanol/Essigester umkristallisiert. Dabei erhält man 1,40 g reines 6-Cyclopropyl-4(3 H)-chinazolinon vom Schmelzpunkt 201—202°.

Beispiel 12

0,395 g Natriumhydrid (57%ige Öldispersion) wird mit Pentan gewaschen und in 25 ml wasserfreiem Dimethylformamid suspendiert. Unter einer Argon-Atmosphäre versetzt man mit 1,40 g 6-Cyclopropyl-4(3 H)-chinazolinon und rührt die Reaktionsmischung während 15 Minuten bei Raumtemperatur und während 30 Minuten bei 50°. Nach Abkühlen auf 30° gibt man 1,0 g Methyl trans-3-Chloracrylat in 5 ml wasserfreiem Dimethylformamid tropfenweise hinzu. Die Reaktionsmischung wird anschließend während 75 Minuten auf 50° erwärmt und im Vakuum eingeengt, um den Großteil des Dimethylformamid zu entfernen. Nach Zugabe von Wasser wird der erhaltene Festkörper abfiltriert und aus Methylenchlorid/Methanol umkristallisiert. Dabei erhält man 1,24 g reinen trans-3-(6-Cyclopropyl-4-oxo-4 H-chinazolin-3-yl)-2-propensäure-methylester vom Schmelzpunkt 178°.

Beispiel 13

Eine Mischung aus 1,18 g trans-3-(6-Cyclopropyl-4-oxo-4 H-chinazolin-3-yl)-2-propensäure-

## Beispiel 13

Eine Mischung aus 1,18 g trans-3-(6-Cyclopropyl-4-oxo-4 H-chinazolin-3-yl)-2-propensäure-methylester und 50 ml 6 N Salzsäure wird während 13 Minuten unter Rühren zum Rückfluß erhitzt. Nach Abkühlen auf Raumtemperatur versetzt man mit 150 ml Wasser und läßt die Mischung in der Kälte stehen. Aus dem abfiltrierten Rohprodukt erhält man nach Umkristallisieren aus Pyridin 0,38 g reine trans-3-(6-Cyclopropyl-4-oxo-4 H-chinazolin-3-yl)-2-propensäure vom Schmelzpunkt 291—293°.

## Beispiel 14

0,367 g Natriumhydrid (57%ige Öldispersion) werden mit Pentan gewaschen und in 25 ml wasserfreiem Dimethylformamid suspendiert. Unter einer Argon-Atmosphäre gibt man 1,23 g 6-Chlor-4(3 H)-chinazolinon hinzu und rührt die Reaktionsmischung während 15 Minuten bei Raumtemperatur und während 30 Minuten bei 50°. Nach Abkühlen auf 30° versetzt man tropfenweise mit 0,91 g Methyl-trans-3-chloracrylat in 5 ml wasserfreiem Dimethylformamid und erwärmt anschließend während 75 Minuten 50°. Im Vakuum entfernt den größten Teil des Dimethylformamids, gibt Wasser hinzu und filtriert den entstandenen Niederschlag ab. Durch Umkristallisieren aus Methylenchlorid/Methanol erhält man 0,95 g reinen trans-3-(6-Chlor-4-oxo-4 H-chinazolin-3-yl)-2-propensäure-methylester vom Schmelzpunkt 171—172°.

## Beispiel 15

Eine Mischung aus 0,92 g trans-3-(6-Chlor-4-oxo-4 H-chinazolin-3-yl)-2-propensäure-methylester und 30 ml 6 N Salzsäure wird unter Rühren während 15 Minuten zum Sieden erhitzt. Nach Abkühlen auf Raumtemperatur versetzt man mit 200 ml Wasser und läßt die Mischung in der Kälte stehen. Nach beendeter Kristallisation wird das Rohprodukt abfiltriert und aus Pyridin umkristallisiert, wobei man 0,31 g reine trans-3-(6-Chlor-4-oxo-4 H-chinazolin-3-yl)-2-propensäure vom Schmelzpunkt 297—298° erhält.

## Beispiel 16

Eine Mischung aus 1,97 g Methyl 5-(Methylthio)-anthranilat und 1,6 ml Formamid wird unter Rühren während 4 Stunden auf 140—145° und anschließend während 4 Stunden auf 190° erwärmt. Nach Abkühlen auf Raumtemperatur wird überschüssiges Formamid im Vakuum entfernt. Den Rückstand versetzt man mit Wasser und filtriert den dabei entstehenden Niederschlag ab. Durch Umkristallisieren aus Methanol/Essigester erhält man 0,73 g reines 6-(Methylthio)-4(3 H)-chinazolinon vom Schmelzpunkt 203—204,5°.

## Beispiel 17

0,205 g Natriumhydrid (57%ige Öldispersion) werden mit Pentan gewaschen und mit 25 ml wasserfreiem Dimethylformamid suspendiert. Unter einer Argon-Atmosphäre gibt man 0,720 g 6-(Methylthio)-4(3 H)-chinazolinon hinzu und rührt die Reaktionsmischung während 10 Minuten bei Raumtemperatur und während 30 Minuten bei 50°. Nach Abkühlen auf 30° versetzt man tropfenweise mit 0,497 g Methyl-trans-3-chloracrylat in 5 ml wasserfreiem Dimethylformamid und erwärmt die Mischung während einer Stunde auf 50°. Anschließend wird der Großteil des Dimethylformamids im Vakuum entfernt, der Rückstand mit Wasser versetzt, und der dabei entstandene Niederschlag abfiltriert. Nach Umkristallisieren aus Methylenchlorid/Methanol resultieren 0,700 g reiner trans-3-(6-Methylthio-4-oxo-4 H-chinazolin-3-yl)-2-propensäure-methylester vom Schmelzpunkt 170°.

## Beispiel 18

Eine Mischung aus 0,65 g trans-3-(6-Methylthio-4-oxo-4 H-chinazolin-3-yl)-2-propensäure-methylester und 30 ml 6 N Salzsäure werden unter Rühren während 20 Minuten zum Rückfluß erhitzt. Nach Abkühlen auf Raumtemperatur versetzt man mit 100 ml Wasser und läßt die Mischung in der Kälte stehen. Das auskristallisierte Rohprodukt wird abfiltriert und aus Pyridin umkristallisiert. Dabei erhält man 0,47 g reine trans-3-(6-Methylthio-4-oxo-4 H-chinazolin-3-yl)-2-propensäure vom Schmelzpunkt 271—272°.

Beispiel 19

Eine Mischung aus 3,90 g 5-Isopropoxy-anthranilsäure und 3,2 ml Formamid wird während 4 Stunden auf 140—145° erwärmt und anschließend auf Raumtemperatur abgekühlt. Nach Entfernen von überschüssigem Formamid im Vakuum wird der Rückstand mit Wasser versetzt. Der Niederschlag wird abfiltriert und aus Methanol umkristallisiert. Dabei erhält man 2,99 g reines 6-Isopropoxy-4(3 H)-chinazolinon vom Schmelzpunkt 206—208°.

Beispiel 20

0,888 g Natriumhydrid (57%ige Öldispersion) werden mit Pentan gewaschen und in 25 ml wasserfreiem Dimethylformamid suspendiert. Unter einer Argon-Atmosphäre gibt man 3,31 g 6-Isopropoxy-4(3 H)-chinazolinon hinzu und rührt die Reaktionsmischung während 10 Minuten bei Raumtemperatur und während 45 Minuten bei 50°. Nach Abkühlen auf 30° versetzt man tropfenweise mit 2,150 g Methyl-trans-3-chloracrylat in 7 ml wasserfreiem Dimethylformamid und erwärmt den Ansatz während einer Stunde auf 50°. Man entfernt den größten Teil Dimethylformamid im Vakuum, versetzt mit Wasser und filtriert den entstandenen Festkörper ab. Nach Umkristallisieren aus Methylenchlorid/Methanol erhält man 4,00 g reinen trans-3-(6-Isopropoxy-4-oxo-4 H-chinazolin-3-yl)-2-propensäure-methylester vom Schmelzpunkt 156,6—157,5°.

Beispiel 21

Eine Mischung aus 1,44 g trans-3-(6-Isopropoxy-4-oxo-4 H-chinazolin-3-yl)-2-propensäure-methylester und 60 ml 6 N Salzsäure wird unter Rühren während 5 Minuten zum Rückfluß erhitzt. Nach Abkühlen auf Raumtemperatur versetzt man mit 200 ml Wasser und läßt die Mischung in der Kälte stehen. Das Rohprodukt wird abfiltriert und aus Essigsäure umkristallisiert. Dabei erhält man 0,37 g reine trans-3-(6-Isopropoxy-4-oxo-4 H-chinazolin-3-yl)-2-propensäure vom Schmelzpunkt 232,5—233,5°.

Beispiel 22

Eine Mischung aus 1,0 g 5-(2-Diäthylamino-äthoxy)-anthranilsäure-dihydrochlorid und 1 ml Formamid wird unter Rühren während 7 Stunden auf 150° erwärmt. Nach Entfernen von überschüssigem Formamid im Vakuum wird der Rückstand mit Methanol/Äther behandelt und ergibt 0,81 g eines Festkörpers. Nach Umkristallisieren aus Isopropylalkohol/Wasser erhält man 0,33 g reines 6-(2-Diäthylamino-äthoxy)-4(3 H)-chinazolinon-hydrochlorid vom Schmelzpunkt 233—234°.

Beispiel 23

0,20 g Natriumhydrid (57%ige Öldispersion) werden mit Hexan gewaschen und in 25 ml wasserfreiem Dimethylformamid suspendiert. Unter einer Argon-Atmosphäre gießt man 0,94 g 6-(2-Diäthylamino-äthoxy)-4(3 H)-chinazolinon hinzu und rührt die Mischung während 15 Minuten bei Raumtemperatur und während einer Stunde bei 50°. Nach Abkühlen auf 30° versetzt man tropfenweise mit 0,48 g Methyl-trans-3-chloracrylat in 5 ml Wasser und erwärmt den Ansatz während einer Stunde und 45 Minuten auf 50°. Man entfernt den größten Teil des Dimethylformamid im Vakuum und versetzt den Rückstand mit Wasser. Durch Abfiltrieren des entstandenen Festkörpers erhält man 1,10 g reinen trans-3-[6-(2-Diäthylamino-äthoxy)-4-oxo-4 H-chinazolin-3-yl]-2-propensäure-methylester vom Schmelzpunkt 131—133°.

Beispiel 24

Eine Mischung aus 1,0454 g trans-3-[6-(2-Diäthylamino-äthoxy)-4-oxo-4 H-chinazolin-3-yl]-2-propensäure-methylester und 25 ml 6 N Salzsäure wird unter Rühren während 20 Minuten zum Rückfluß erhitzt. Nach Einengen im Vakuum wird der erhaltene gelbe Festkörper aus Isopropylalkohol/Wasser umkristallisiert. Dabei erhält man 0,69 g reines trans-3-[6-(2-Diäthylamino-äthoxy)-4-oxo-4 H-chinazolin-3-yl]-2-propensäure-hydrochlorid vom Schmelzpunkt 227—230°.

Beispiel 25

Eine Mischung aus 1,74 g 5-(2-Hydroxyäthoxy)-anthranilsäure und 2 ml Formamid wird unter Rühren während 7,5 Stunden auf 150° erwärmt. Nach Zugabe von Wasser wird das Rohprodukt abfiltriert und aus Methanol umkristallisiert. Dabei erhält man 1,24 g reines 6-(2-Hydroxyäthoxy)-4(3 H)-chinazolinon vom Schmelzpunkt 200—202°.

Beispiel 26

Zu 2,0479 g 6-(2-Hydroxyäthoxy)-4(3 H)-chinazolinon in 60 ml wasserfreiem Dimethylformamid gibt man 1,52 ml Diäthylamin und anschließend tropfenweise 1,40 ml Trimethylsilylchlorid. Man rührt während 1,5 Stunden bei 25° und filtriert anschließend vom ausgefallenen Triäthylaminhydrochlorid ab. Das Filtrat wird anschließend zu 0,643 g Natriumhydrid (54%ige Öldispersion), das vorher mit Hexan gewaschen wurde, gegeben. Man rührt während 5 Minuten unter einer Argon-Atmosphäre und erwärmt anschließend die Reaktionsmischung während 45 Minuten auf 50°. Nach Abkühlen auf 30° versetzt man tropfenweise mit 1,356 g Methyl-trans-3-chloracrylat in 2 ml Dimethylformamid und erwärmt die Mischung während 75 Minuten auf 50°. Der größte Teil des Dimethylformamid wird anschließend im Vakuum entfernt. Der Rückstand wird mit 50 ml 6 N Salzsäure versetzt und während 15 Minuten unter Rückfluß zum Sieden erhitzt. Nach dem Abkühlen wird der erhaltene Festkörper abfiltriert, mit Wasser gründlich gewaschen und getrocknet. Dabei erhält man 0,09607 g reine trans-3-[6-(2-Hydroxyäthoxy)-4-oxo-4 H-chinazolin-3-yl]-2-propensäure vom Schmelzpunkt 247—248°.

Beispiel 27

Eine Suspension von 1,60 g trans-3-(6-Isopropyl-4-oxo-4 H-chinazolin-3-yl)-2-propensäure in 20 ml Thionylchlorid wird unter Rühren während 3 Stunden zum Rückfluß erhitzt. Anschließend engt man die Reaktionsmischung im Vakuum ein, gibt Toluol hinzu und dampft erneut im Vakuum ein, um Spuren von Thionylchlorid zu entfernen. Das so erhaltene Säurechlorid wird in 50 ml Toluol gelöst und tropfenweise mit 1,08 g 2-(Diäthylamino)-äthylamin in 15 ml Toluol versetzt. Die Reaktionsmischung wird während 4 Stunden zum Rückfluß erhitzt und dann im Vakuum eingedampft. Man verteilt den Rückstand zwischen Essigester und gesättigter Natriumbicarbonatlösung, trennt die organische Schicht ab, trocknet sie über Magnesiumsulfat und dampft ein. Das erhaltene dunkle Öl kristallisiert aus Hexan und ergibt einen grauen Festkörper, der in 10 ml Methylenchlorid gelöst wird und mit 2 ml 4 N Salzsäure in Methanol behandelt wird. Nach Einengen der Lösung im Vakuum wird der Rückstand aus Isopropylalkohol/Äther kristallisiert. Man erhält dabei 0,90 g trans-3-(6-Isopropyl-4-oxo-4 H-chinazolin-3-yl)-2-propensäure-2-diäthylamino)-äthylamid-hydrochlorid vom Schmelzpunkt 179—180°.

Beispiel 28

Eine Suspension von 1,55 g trans-3-(6-Isopropyl-4-oxo-4 H-chinazolin-3-yl)-2-propensäure in 100 ml Isopropylalkohol wird unter Rühren zum Rückfluß erhitzt und tropfenweise mit 2,71 g 2-(Diäthylamino)-äthylchlorid in 30 ml Isopropylalkohol versetzt. Der Ansatz wird 8 Stunden unter Rühren zum Rückfluß erhitzt. Der ausgefallene Festkörper wird anschließend abfiltriert und mit Isopropylalkohol gut ausgewaschen. Das Filtrat wird im Wasser zur Trockene eingedampft und der dabei anfallene Rückstand zwischen Chloroform und gesättigter Natriumbicarbonatlösung verteilt. Die Chloroformphase wird über Magnesiumsulfat getrocknet und im Vakuum eingedampft. Man erhält 2,5 g eines Öles, das man in 35 ml Methylenchlorid auflöst und mit 4,0 ml 1,77 N Salzsäure in Methanol versetzt. Man gibt Äther hinzu bis zur bleibenden Trübung und läßt kristallisieren. Man erhält so 1,88 g trans-3-(6-Isopropyl-4-oxo-4 H-chinazolin-3-yl)-2-propensäure-2-(diäthylamino)-äthylester-hydrochlorid vom Schmelzpunkt 167—168°.

Beispiel 29

Eine gerührte Lösung von 4,00 g trans-3-Chloracrylsäurechlorid in 10 ml Methylenchlorid wird tropfenweise mit 3,78 g 2-(Diäthylamino)-äthanol in 5 ml Methylenchlorid versetzt. Die Lösung wird 3 Tage bei Raumtemperatur gerührt und anschließend mit gesättigter Natriumbicarbonatlösung gewaschen. Nach Trocknen der organischen Phase über Magnesiumsulfat und Eindampfen im Vakuum wird das erhaltene Öl durch Destillation bei 110—130° (1 mm) gereinigt. Es resultieren 1,63 g trans-3-Chloracrylsäure-2-(diäthylamino)-äthylester.

## Beispiel 30

0,274 g Natriumhydrid (57%ige Öldispersion) werden mit Pentan gewaschen und anschließend in 10 ml wasserfreiem Dimethylformamid suspendiert. Unter einer Argon-Atmosphäre gibt man 0,941 g 6-Isopropyl-4(3 H)-chinazolinon hinzu und erwärmt die Mischung unter Rühren 30 Minuten lang auf 50°. Nach Abkühlen auf Raumtemperatur versetzt man tropfenweise mit 0,663 g Methyl-cis-chloracrylat in 5 ml Dimethylformamid. Die Reaktionsmischung wird unter Rühren 75 Minuten auf 50° erwärmt und im Vakuum eingeengt und den größten Teil des Dimethylformamids zu entfernen. Nach Zugabe von Wasser wird der ausgefallene Festkörper aus Methylenchlorid/Methanol umkristallisiert. Man erhält dabei 0,58 g trans-(6-Isopropyl-4-oxo-4 H-chinazolin-3-yl)-2-propensäure-methylester vom Schmelzpunkt 144—145°.

## Beispiel 31

Eine Lösung von 2,726 g 6-Hydroxy-4(3 H)-chinazolinon in 110 ml wasserfreiem Dimethylformamid versetzt man mit 2,6 ml Triäthylamin, gefolgt von 2,6 ml Trimethylchlorsilan. Man rührt 2 Stunden bei Raumtemperatur und filtriert anschließend vom ausgefallenen Triäthylaminhydrochlorid ab. Das Filtrat wird in einer Portion zu 1,06 g Natriumhydrid (57%ige Öldispersion), das vorher mit Pentan gewaschen wurde, gegeben. Der Ansatz wird unter Rühren 90 Minuten auf 50° erwärmt, auf Raumtemperatur abgekühlt und tropfenweise mit 2,02 g Methyl-trans-3-chloracrylat in 10 ml Dimethylformamid versetzt. Nach weiteren 2 Stunden bei 50° entfernt man das Dimethylformamid im Vakuum, versetzt mit Wasser und filtriert den so erhaltenen Festkörper ab. Durch Umkristallisieren aus Essigester/Methanol erhält man 2,725 g trans-3-(6-Hydroxy-4-oxo-4 H-chinazolin-3-yl)-2-propensäure-methylester vom Schmelzpunkt 252—256°.

## Beispiel 32

Eine Mischung aus 8,35 g trans-3-(6-Hydroxy-4-oxo-4 H-chinazolin-3-yl)-2-propensäure-methylester und 500 ml 6 N Salzsäure wird unter Rühren 15 Minuten zum Rückfluß erhitzt. Nach dem Abkühlen versetzt man mit Wasser und filtriert den ausgefallenen Festkörper ab. Man erhält dabei 6,33 g trans-3-(6-Hydroxy-4-oxo-4 H-chinazolin-3-yl)-2-propensäure vom Schmelzpunkt 294—296°.

## Beispiel 33

Eine gerührte Suspension von 1,823 g trans-3-(6-Methylthio-4-oxo-4 H-chinazolin-3-yl)-2-propensäure in 80 ml Wasser und 7,7 ml 1 N Natriumhydroxid wird bei Raumtemperatur mit 1,827 g Natriummetaperiodat versetzt. Man rührt 4,5 Stunden bei Raumtemperatur, gibt 5 ml Essigsäure hinzu und filtriert 15 Minuten später den ausgefallenen Festkörper ab. Das Rohprodukt wird zweimal aus Essigsäure umkristallisiert und ergibt 1,102 g reine trans-3-(6-Methylsulfinyl-4-oxo-4 H-chinazolin-3-yl)-2-propensäure vom Schmelzpunkt 275—276°.

## Beispiel 34

Eine gerührte Suspension von 1,8845 g trans-3-(6-Methylthio-4-oxo-4 H-chinazolin-3-yl)-2-propensäure in 100 ml Essigsäure wird mit 1,63 ml 30%igem Wasserstoffperoxid versetzt. Man rührt die Reaktionsmischung 3 Stunden bei 50°, kühlt ab auf Raumtemperatur und filtriert den ausgefallenen Festkörper ab. Umkristallisieren aus Essigsäure liefert 0,8097 g reine trans-3-(6-Methylsulfonyl-4-oxo-4 H-chinazolin-3-yl)-2-propensäure vom Schmelzpunkt 284—290°.

## Beispiel 35

Eine Lösung von 1,8120 g trans-3-(6-Isopropyl-4-oxo-4 H-chinazolin-3-yl)-2-propensäure in 30 ml Thionylchlorid wird unter Rühren 2 Stunden zum Rückfluß erhitzt. Man dampft anschließend im Vakuum ein und entfernt Spuren von Thionylchlorid durch Abdampfen mit trockenem Toluol. Das erhaltene feste Säurechlorid wird bei Raumtemperatur mit 30 ml wasserfreiem Dimethylformamid versetzt, gefolgt von 2,4 ml 2-(Diisopropylamino)-äthylamin. Man rührt 2 Stunden und läßt anschließend 16 Stunden bei Raumtemperatur stehen. Die Reaktionsmischung wird im Hochvakuum zu einem dunklen Öl eingedampft, das man in 20 ml Methanol aufnimmt und mit 1,6 ml 4,4 N Salzsäure in Methanol behandelt. Nach Entfernen des Methanols im Vakuum wird der Rückstand mit Isopropylalkohol/Äther behandelt. Durch Umkristallisieren des erhaltenen Festkörpers aus Methylenchlorid/

Äther erhält man 1,3037 g reines trans-N-[(2-Diisopropylamino)-äthyl]-3-(6-isopropyl-4-oxo-4H-chinazolin-3-yl)-2-propenamid-hydrochlorid vom Schmelzpunkt 209—213°.

### Beispiel A

Tabletten der nachfolgenden Zusammensetzung werden hergestellt:

| | mg/Tablette | mg/Tablette | mg/Tablette |
|---|---|---|---|
| Trans-3-(6-Isopropyl-4-oxo-4H-chinazolin-3-yl)-2-propensäure | 1 | 5 | 25 |
| Lactose | 221 | 217 | 197 |
| Mikrokristalline Zellulose | 45 | 45 | 45 |
| Modifizierte Stärke | 30 | 30 | 30 |
| Magnesiumstearat | 3 | 3 | 3 |
| | 300 mg | 300 mg | 300 mg |

### Verfahren

Der Wirkstoff wird mit einer äquivalenten Menge Lactose gut vermischt. Man versetzt mit einer mikrokristallinen Cellulose, der modifizierten Stärke und den Rest der Lactose und vermischt gut. Anschließend gibt man das Magnesiumstearat hinzu, mischt während 3 Minuten und verpreßt die Mischung auf einer geeigneten Tablettenpresse.

### Beispiel B

Tabletten der nachfolgenden Zusammensetzung werden hergestellt:

| | mg/Tablette | mg/Tablette | mg/Tablette |
|---|---|---|---|
| Trans-3-(6-Isopropyl-4-oxo-4H-chinazolin-3-yl)-2-propensäure | 1 | 5 | 25 |
| Lactose | 202 | 198 | 223 |
| Modifizierte Stärke | 25 | 25 | 30 |
| Vorgelatinisierte Stärke | 20 | 20 | 20 |
| Destilliertes Wasser | — | — | — |
| Magnesiumstearat | 2 | 2 | 2 |
| | 250 mg | 250 mg | 300 mg |

### Verfahren

Die ersten vier Bestandteile werden in einem geeigneten Mischer vermischt. Es wird mit genügend Wasser granuliert und in einem geeigneten großen Ofen getrocknet. Man gibt das Magnesiumstearat hinzu, mischt gut und verpreßt auf einer geeigneten Tablettenpresse.

Beispiel C

Kapseln der folgenden Zusammensetzung werden hergestellt:

| | mg/Kapsel | mg/Kapsel | mg/Kapsel |
|---|---|---|---|
| Trans-3-(6-Isopropyl-4-oxo-4H-chinazolin-3-yl)-2-propensäure | 1 | 5 | 25 |
| Lactose | 253 | 249 | 224 |
| Stärke | 30 | 30 | 30 |
| Talk | 15 | 15 | 15 |
| Magnesiumstearat | 1 | 1 | 1 |
| | 300 mg | 300 mg | 300 mg |

Verfahren

Die ersten drei Bestandteile werden in einem geeigneten Mischer vermischt. Man gibt Talk und Magnesiumstearat hinzu, mischt kurz und füllt das Ganze in einer geeigneten Kapselabfüllmaschine ab.

Beispiel D

Tabletten der nachfolgenden Zusammensetzung werden hergestellt:

| | mg/Tablette | mg/Tablette | mg/Tablette |
|---|---|---|---|
| Trans-3-(6-Methylthio-4-oxo-4H-chinazolin-3-yl)-2-propensäure | 1 | 5 | 25 |
| Lactose | 202 | 198 | 223 |
| Modifizierte Stärke | 25 | 25 | 30 |
| Vorgelatinisierte Stärke | 20 | 20 | 20 |
| Destilliertes Wasser q. s. | – | – | – |
| Magnesiumstearat | 2 | 2 | 2 |
| | 250 mg | 250 mg | 300 mg |

Verfahren

Die ersten vier Bestandteile werden in einem geeigneten Mischer vermischt. Es wird mit genügend Wasser granuliert und in einem geeigneten Ofen getrocknet. Man gibt das Magnesiumstearat hinzu, mischt gut und verpreßt auf einer geeigneten Tablettenpresse.

Beispiel E

Kapseln der folgenden Zusammensetzung werden hergestellt:

| | mg/Kapsel | mg/Kapsel | mg/Kapsel |
|---|---|---|---|
| Trans-3-(6-Methylthio-4-oxo-4 H-chinazolin-3-yl)-2-propensäure | 1 | 5 | 25 |
| Lactose | 253 | 249 | 229 |
| Stärke | 30 | 30 | 30 |
| Talk | 15 | 15 | 15 |
| Magnesiumstearat | 1 | 1 | 1 |
| | 300 mg | 300 mg | 300 mg |

Verfahren

Die ersten drei Bestandteile werden in einem geeigneten Mischer vermischt. Man gibt Talk und Magnesiumstearat hinzu, mischt kurz und füllt das Ganze in einer geeigneten Kapselabfüllmaschine ab.

Beispiel F

Tabletten der nachfolgenden Zusammensetzung werden hergestellt:

| | mg/Tablette | mg/Tablette | mg/Tablette |
|---|---|---|---|
| Trans-3-(6-Methylthio-4-oxo-4 H-chinazolin-3-yl)-2-propensäure | 1 | 5 | 25 |
| Lactose | 221 | 217 | 197 |
| Mikrokristalline Zellulose | 45 | 45 | 45 |
| Modifizierte Stärke | 30 | 30 | 30 |
| Magnesiumstearat | 3 | 3 | 3 |
| | 300 mg | 300 mg | 300 mg |

Verfahren

Der Wirkstoff wird mit einer äquivalenten Menge Lactose gut vermischt. Man versetzt mit der mikrokristallinen Cellulose, der modifizierten Stärke und den Rest der Lactose und vermischt gut. Anschließend gibt man das Magnesiumstearat hinzu, mischt während 3 Minuten und verpreßt die Mischung auf einer geeigneten Tablettenpresse.

**Patentansprüche für die Vertragsstaaten: BE, CH, DE, FR, GB, IT, LI, LU, NL, SE**

1. Trans-Chinazolinderivate der allgemeinen Formel I

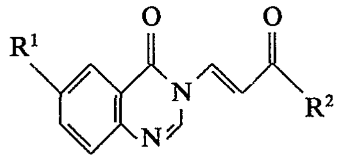

(I)

worin $R^1$ $(C_{1-7})$-Alkyl, $(C_{3-7})$-Cycloalkyl, $(C_{1-7})$-Alkoxy, Hydroxy, Halogen, $(C_{1-7})$-Alkylthio, $(C_{1-7})$-Alkylsulfinyl, $(C_{1-7})$-Alkylsulfonyl, di-$(C_1—C_7)$Alkyl-$N(CH_2)_n$—O—, Hydroxy-$(C_{1-7})$-Alkoxy, Trifluormethyl, Nitro, Amino, mono-$(C_{1-7})$-Alkylamino oder di-$(C_{1-7})$-Alkylamino, $R^2$ Hydroxy, $(C_{1-7})$-Alkoxy, di-$(C_1—C_7)$Alkyl-$N(CH_2)_n$—O— oder di-$(C_1—C_7)$Alkyl-$N(CH_2)_n$—NH— und n eine ganze Zahl von 2—7 bedeuten,

und wenn $R^2$ Hydroxy bedeutet, Salze davon mit pharmazeutisch annehmbaren Basen, oder wenn $R^1$ Amino, mono-$(C_{1-7})$-Alkylamino, di-$(C_{1-7})$-Alkylamino oder di-$(C_1—C_7)$Alkyl—$N(CH_2)_n$—O— und/oder $R^2$ di-$(C_1—C_7)$Alkyl—$N(CH_2)_n$—O— oder di-$(C_1—C_7)$Alkyl—$N(CH_2)_n$—NH— bedeuten, Salze davon mit pharmazeutisch annehmbaren Säuren.

2. Verbindungen gemäß Anspruch 1 worin $R^1$ $(C_{1-7})$-Alkyl, $(C_{3-7})$-Cycloalkyl, $(C_{1-7})$-Alkoxy, Hydroxy, Halogen, $(C_{1-7})$-Alkylthio, di-$(C_1—C_7)$Alkyl-$N(CH_2)_n$—O— oder 2-Hydroxy-äthoxy bedeutet und n die in Anspruch 1 angegebene Bedeutung besitzt.

3. Verbindungen gemäß Anspruch 1 worin $R^1$ $(C_{1-7})$-Alkylsulfinyl oder $(C_{1-7})$-Alkylsulfonyl bedeutet.

4. Verbindungen gemäß Anspruch 2 worin $R^1$ $(C_{1-7})$-Alkyl, $(C_{1-7})$-Cycloalkyl, $(C_{1-7})$-Alkoxy oder $(C_{1-7})$-Alkylthio bedeutet.

5. Verbindungen gemäß Anspruch 2 worin $R^1$ $(C_{1-7})$-Alkyl oder $(C_{1-7})$-Alkylthio und $R^2$ Hydroxy oder di-$(C_1—C_7)$Alkyl-$N(CH_2)_n$—NH— bedeuten, und n die in Anspruch 1 angegebene Bedeutung besitzt.

6. Verbindungen gemäß Anspruch 2 worin $R^1$ Hydroxy oder $(C_{1-7})$-Alkoxy und $R^2$ Hydroxy bedeuten.

7. Verbindungen gemäß Anspruch 3 worin $R^1$ $(C_{1-7})$-Alkylsulfinyl und $R^2$ Hydroxy bedeuten.

8. Verbindungen gemäß einem der Ansprüche 1—4 worin $R^2$ Hydroxy bedeutet.

9. trans-3-(6-Isopropyl-4-oxo-4 H-chinazolin-3-yl)-2-propensäure.

10. trans-3-(6-Methylthio-4-oxo-4 H-chinazolin-3-yl)-2-propensäure.

11. trans-3-(6-Isopropyl-4-oxo-4 H-chinazolin-3-yl)-2-propensäure-2-(diäthylamino)-äthylamid-hydrochlorid.

12. trans-3-(6-Isopropoxy-4-oxo-4 H-chinazolin-3-yl)-2-propensäure.

13. trans-3-(6-Hydroxy-4-oxo-4 H-chinazolin-3-yl)-2-propensäure.

14. trans-3-(6-Methylsulfinyl-4-oxo-4 H-chinazolin-3-yl)-2-propensäure.

15. Trans-chinazoline der allgemeinen Formel VII

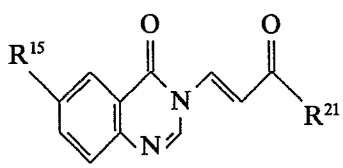

(VII)

worin $R^{15}$ eine geschützte Amino-, Mono-$(C_{1-7})$-Alkylamino-, Hydroxy- oder Hydroxy-$(C_{1-7})$-Alkoxy-Gruppe und $R^{21}$ $(C_{1-7})$-Alkoxy, di-$(C_1—C_7)$Alkyl-$N(CH_2)_n$—O— oder di-$(C_1—C_7)$Alkyl-$N(CH_2)_n$—NH— bedeuten.

16. Verbindungen gemäß einem der Ansprüche 1—14, als pharmazeutische Wirkstoffe.

17. trans-3-(6-Methylthio-4-oxo-4 H-chinazolin-3-yl)-2-propensäure als pharmazeutischer Wirkstoff.

18. Verbindungen gemäß einem der Ansprüche 1—14 als anti-allergische Wirkstoffe.

19. trans-3-(6-Methylthio-4-oxo-4 H-chinazolin-3-yl)-2-propensäure als anti-allergischer Wirkstoff.

20. Verfahren zur Herstellung von trans-3-(4-Oxo-4 H-chinazolin-3-yl)-2-propensäure-Derivaten der allgemeinen Formel I, gemäß Anspruch 1, und ihrer pharmazeutisch annehmbaren Salze, dadurch gekennzeichnet, daß man

a) zur Herstellung von Verbindungen der allgemeinen Formel I a

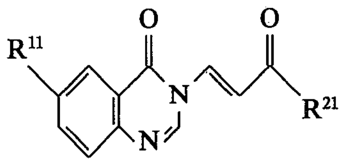

(Ia)

worin $R^{11}$ $(C_{1-7})$-Alkyl, $(C_{3-7})$-Cycloalkyl, $(C_{1-7})$-Alkoxy, Nitro, Halogen, $(C_{1-7})$-Alkylthio, $(C_{1-7})$-Alkylsulfinyl, $(C_{1-7})$-Alkylsulfonyl, Trifluormethyl, di-$(C_{1-7})$-Alkylamino oder di-$(C_1{-}C_7)$Alkyl-$N(CH_2)_n{-}O{-}$, $R^{21}$ $(C_{1-7})$-Alkoxy, di-$(C_1{-}C_7)$Alkyl-$N(CH_2)_n{-}O{-}$ oder di-$(C_1{-}C_7)$Alkyl-$N(CH_2)_n{-}NH$ und n eine ganze Zahl von 2—7 bedeuten, eine Verbindung der allgemeinen Formel II

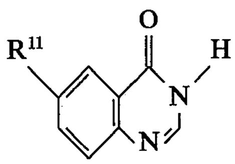

(II)

worin $R^{11}$ obige Bedeutung besitzt,
mit einem 3-Chloracrylsäure-Derivat der allgemeinen Formel III

$$ClCH{=}CH{-}\overset{\overset{O}{\|}}{C}{-}R^{21} \qquad (III)$$

worin $R^{21}$ obige Bedeutung besitzt,
umsetzt, oder

b) zur Herstellung von Verbindungen der allgemeinen Formel Ib

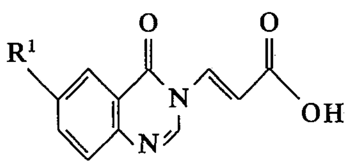

(Ib)

worin $R^1$ obige Bedeutung besitzt,
eine Verbindung der allgemeinen Formel Ic

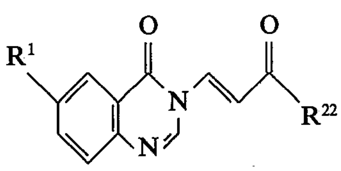

(Ic)

worin $R^{22}$ $(C_{1-7})$-Alkoxy bedeutet und $R^1$ obige Bedeutung besitzt,
hydrolysiert, oder

c) zur Herstellung von Verbindungen der allgemeinen Formel Id

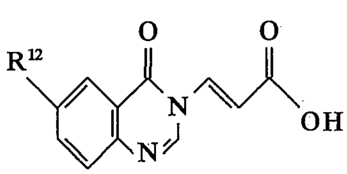

(Id)

worin $R^{12}$ $(C_{1-7})$-Alkylsulfinyl oder $(C_{1-7})$-Alkylsulfonyl bedeutet,
eine Verbindung der allgemeinen Formel Ie

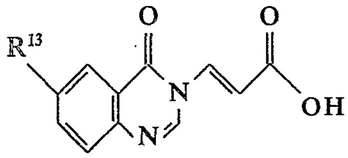

(Ie)

worin $R^{13}$ $(C_{1-7})$-Alkylthio bedeutet, oxydiert, oder

d) zur Herstellung von Verbindungen der allgemeinen Formel If

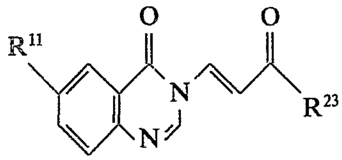

(If)

worin $R^{23}$ di-$(C_1-C_7)$Alkyl$-N(CH_2)_n-O-$ oder di-$(C_1-C_7)$Alkyl$-N(CH_2)_n-NH-$ bedeutet und $R^{11}$ und n obige Bedeutung besitzen, ein aktives Derivat einer Carbonsäure der allgemeinen Formel Ig

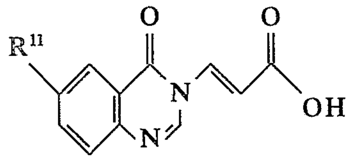

(Ig)

worin $R^{11}$ obige Bedeutung besitzt, mit einem Alkanol der allgemeinen Formel IV

$$\text{di-}(C_1-C_7)\text{Alkyl}-N(CH_2)_n-OH \quad (IV)$$

oder mit einem Amin der allgemeinen Formel V

$$\text{di-}(C_1-C_7)\text{Alkyl}-N(CH_2)_n-NH_2 \quad (V)$$

worin n jeweils obige Bedeutung besitzt, behandelt, oder

e) zur Herstellung von Verbindungen der allgemeinen Formel Ih

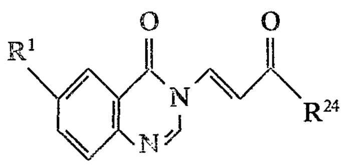

(Ih)

worin $R^{24}$ di-$(C_1-C_7)$Alkyl$-N(CH_2)_n-O-$ bedeutet und $R^1$ und n obige Bedeutung besitzen, eine Carbonsäure der obigen allgemeinen Formel Ib mit einem Halogenid der allgemeinen Formel VI

$$\text{di-}(C_1-C_7)\text{Alkyl}-N(CH_2)_n-X \quad (VI)$$

worin X Halogen bedeutet und n obige Bedeutung besitzt, behandelt, oder

f) zur Herstellung von Verbindungen der allgemeinen Formel Ii

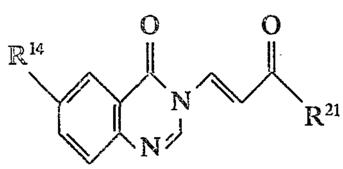

(Ii)

worin $R^{14}$ Amino, mono-$(C_{1-7})$-Alkylamino, Hydroxy oder Hydroxy-$(C_{1-7})$-Alkoxy bedeutet und $R^{21}$ obige Bedeutung besitzt,
in einer Verbindung der allgemeinen Formel VII

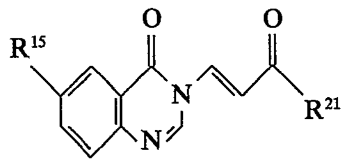

(VII)

worin $R^{15}$ eine geschützte Amino-, mono-$(C_{1-7})$-Alkylamino-, Hydroxy- oder Hydroxy-$(C_{1-7})$-Alkoxygruppe bedeutet und $R^{21}$ obige Bedeutung besitzt,
die Schutzgruppe abspaltet, und

g) erwünschtenfalls eine Verbindung der allgemeinen Formel I, worin $R^2$ Hydroxy bedeutet, in ein pharmazeutisch annehmbares Salz mit einer Base überführt, oder eine Verbindung der allgemeinen Formel I, worin $R^1$ Amino, mono-$(C_{1-7})$-Alkylamino, di-$(C_{1-7})$-Alkylamino oder di-$(C_1-C_7)$-Alkyl$-N(CH_2)_n-O-$ bedeutet und/oder $R^2$ di-$(C_1-C_7)$Alkyl$-N(CH_2)_n-O-$ oder di-$(C_1-C_7)$-Alkyl$-N(CH_2)_n-NH-$ bedeutet und n obige Bedeutung besitzt, in ein pharmazeutisch annehmbares Säureadditionssalz überführt.

21. Arzneimittel, enthaltend eine Verbindung der in Anspruch 1 angegebenen allgemeinen Formel I oder ein pharmazeutisch annehmbares Salz davon.

22. Arzneimittel, enthaltend trans-3-(6-Methylthio-4-oxo-4 H-chinazolin-3-yl)-2-propensäure oder ein pharmazeutisch annehmbares Salz davon.

23. Anti-allergische Mittel, enthaltend eine Verbindung der in Anspruch 1 angegebenen allgemeinen Formel I oder ein pharmazeutisch annehmbares Salz davon.

24. Anti-allergische Mittel, enthaltend trans-3-(6-Methylthio-4-oxo-4 H-chinazolin-3-yl)-2-propensäure oder ein pharmazeutisch annehmbares Salz davon.

**Patentansprüche für den Vertragsstaat: AT**

1. Verfahren zur Herstellung von trans-Chinazolinderivaten der allgemeinen Formel I

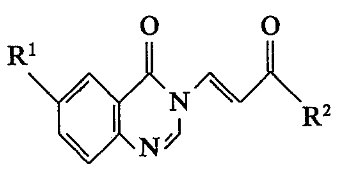

(I)

worin $R^1$ $(C_{1-7})$-Alkyl, $(C_{3-7})$-Cycloalkyl, $(C_{1-7})$-Alkoxy, Hydroxy, Halogen, $(C_{1-7})$-Alkylthio, $(C_{1-7})$-Alkylsulfinyl, $(C_{1-7})$-Alkylsulfonyl, di-$(C_1-C_7)$Alkyl$-N(CH_2)_n-O-$, Hydroxy-$(C_{1-7})$-Alkoxy, Trifluormethyl, Nitro, Amino, mono-$(C_{1-7})$-Alkylamino oder di-$(C_{1-7})$-Alkylamino, $R^2$ Hydroxy, $(C_{1-7})$-Alkoxy, di-$(C_1-C_7)$Alkyl$-N(CH_2)_n-O-$ oder di-$(C_1-C_7)$Alkyl$-N(CH_2)_n-NH-$ und n eine ganze Zahl von 2—7 bedeuten,

und wenn $R^2$ Hydroxy bedeutet, Salze davon mit pharmazeutisch annehmbaren Basen, oder wenn $R^1$ Amino, mono-$(C_{1-7})$-Alkylamino, di-$(C_{1-7})$-Alkylamino oder di-$(C_1-C_7)$Alkyl$-N(CH_2)_n-O-$ und/oder $R^2$ di-$(C_1-C_7)$Alkyl$-N(CH_2)_n-O-$ oder di-$(C_1-C_7)$Alkyl$-N(CH_2)_n-NH-$ bedeuten, Salze davon mit pharmazeutisch annehmbaren Säuren, dadurch gekennzeichnet, daß man

a) zur Herstellung von Verbindungen der allgemeinen Formel I a

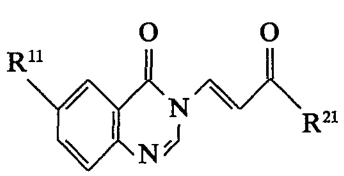

(Ia)

worin $R^{11}$ $(C_{1-7})$-Alkyl, $(C_{3-7})$-Cycloalkyl, $(C_{1-7})$-Alkoxy, Nitro, Halogen, $(C_{1-7})$-Alkylthio, $(C_{1-7})$-Alkylsulfinyl, $(C_{1-7})$-Alkylsulfonyl, Trifluormethyl, di-$(C_{1-7})$-Alkylamino oder di-$(C_1-C_7)$Alkyl-$N(CH_2)_n-O-$, $R^{21}$ $(C_{1-7})$-Alkoxy, di-$(C_1-C_7)$Alkyl$-N(CH_2)_n-O-$ oder di-$(C_1-C_7)$Alkyl$-N(CH_2)_n-NH$

und n eine ganze Zahi von 2–7 bedeuten, eine Verbindung der allgemeinen Formel II

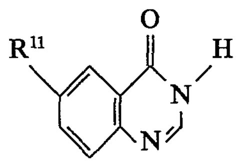

(II)

worin $R^{11}$ obige Bedeutung besitzt,
mit einem 3-Chloracrylsäure-Derivat der allgemeinen Formel III

$$ClCH{=}CH{-}\overset{\overset{O}{\|}}{C}{-}R^{21}$$ (III)

worin $R^{21}$ obige Bedeutung besitzt,
umsetzt, oder

b) zur Herstellung von Verbindungen der allgemeinen Formel I b

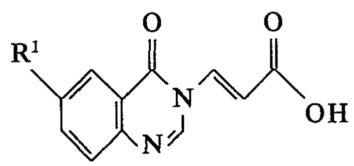

(Ib)

worin $R^1$ obige Bedeutung besitzt,
eine Verbindung der allgemeinen Formel I c

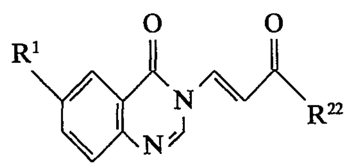

(Ic)

worin $R^{22}$ ($C_{1-7}$)-Alkoxy bedeutet und $R^1$ obige Bedeutung besitzt,
hydrolysiert, oder

c) zur Herstellung von Verbindungen der allgemeinen Formel I d

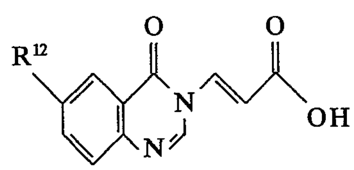

(Id)

worin $R^{12}$ ($C_{1-7}$)-Alkylsulfinyl oder ($C_{1-7}$)-Alkylsulfonyl bedeutet,
eine Verbindung der allgemeinen Formel I e

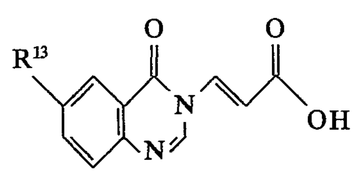

(Ie)

worin $R^{13}$ ($C_{1-7}$)-Alkylthio bedeutet,
oxydiert, oder

d) zur Herstellung von Verbindungen der allgemeinen Formel I f

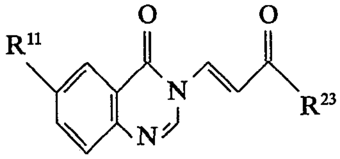

(If)

worin $R^{23}$ di-($C_1$—$C_7$)Alkyl—$N(CH_2)_n$—O— oder di-($C_1$—$C_7$)Alkyl—$N(CH_2)_n$—NH— bedeutet und $R^{11}$ und n obige Bedeutung besitzen,
ein aktives Derivat einer Carbonsäure der allgemeinen Formel I g

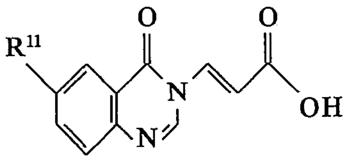

(Ig)

worin $R^{11}$ obige Bedeutung besitzt,
mit einem Alkanol der allgemeinen Formel IV

$$\text{di-}(C_1\text{—}C_7)\text{Alkyl—}N(CH_2)_n\text{—}OH \quad \text{(IV)}$$

oder mit einem Amin der allgemeinen Formel V

$$\text{di-}(C_1\text{—}C_7)\text{Alkyl—}N(CH_2)_n\text{—}NH_2 \quad \text{(V)}$$

worin n jeweils obige Bedeutung besitzt,
behandelt, oder

e) zur Herstellung von Verbindungen der allgemeinen Formel I h

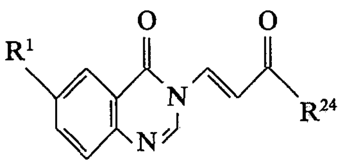

(Ih)

worin $R^{24}$ di-($C_1$—$C_7$)Alkyl—$N(CH_2)_n$—O— bedeutet und $R^1$ und n obige Bedeutung besitzen, eine Carbonsäure der obigen allgemeinen Formel I b mit einem Halogenid der allgemeinen Formel VI

$$\text{di-}(C_1\text{—}C_7)\text{Alkyl—}N(CH_2)_n\text{—}X \quad \text{(VI)}$$

worin X Halogen bedeutet und n obige Bedeutung besitzt,
behandelt, oder

f) zur Herstellung von Verbindungen der allgemeinen Formel I i

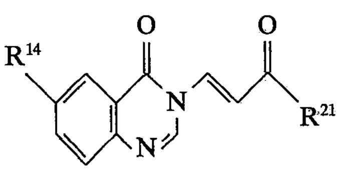

(Ii)

worin $R^{14}$ Amino, mono-($C_{1-7}$)-Alkylamino, Hydroxy oder Hydroxy-($C_{1-7}$)-Alkoxy bedeutet und $R^{21}$ obige Bedeutung besitzt,
in einer Verbindung der allgemeinen Formel VII

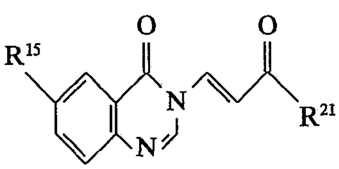

(VII)

worin $R^{15}$ eine geschützte Amino-, mono-$(C_{1-7})$-Alkylamino-, Hydroxy- oder Hydroxy-$(C_{1-7})$-Alkoxygruppe bedeutet und $R^{21}$ obige Bedeutung besitzt,
die Schutzgruppe abspaltet, und

g) erwünschtenfalls eine Verbindung der allgemeinen Formel I, worin $R^2$ Hydroxy bedeutet, in ein pharmazeutisch annehmbares Salz mit einer Base überführt, oder eine Verbindung der allgemeinen Formel I, worin $R^1$ Amino, mono-$(C_{1-7})$-Alkylamino, di-$(C_{1-7})$-$(C_{1-7})$-Alkylamino oder di-$(C_1-C_7)$Alkyl$-N(CH_2)_n-O-$ bedeutet und/oder $R^2$ di-$(C_1-C_7)$Alkyl$-N(CH_2)_n-O-$ oder di-$(C_1-C_7)$Alkyl$-N(CH_2)_n-NH-$ bedeutet und n obige Bedeutung besitzt, in ein pharmazeutisch annehmbares Säureadditionssalz überführt.

2. Verfahren gemäß Anspruch 1, dadurch gekennzeichnet, daß $R^1$ $(C_{1-7})$-Alkyl, $(C_{1-7})$-Cycloalkyl, $(C_{1-7})$-Alkoxy, Hydroxy, Halogen, $(C_{1-7})$-Alkylthio, di-$(C_1-C_7)N(CH_2)_n-O-$ oder 2-Hydroxyäthoxy bedeutet und n die in Anspruch 1 angegebene Bedeutung besitzt.

3. Verfahren gemäß Anspruch 1, dadurch gekennzeichnet, daß $R^1$ $(C_{1-7})$-Alkylsulfinyl oder $(C_{1-7})$-Alkylsulfonyl bedeutet.

4. Verfahren gemäß Anspruch 2, dadurch gekennzeichnet, daß $R^1$ $(C_{1-7})$-Alkyl, $(C_{1-7})$-Cycloalkyl, $(C_{1-7})$-Alkoxy oder $(C_{1-7})$-Alkylthio bedeutet.

5. Verfahren gemäß Anspruch 2, dadurch gekennzeichnet, daß $R^1$ $(C_{1-7})$-Alkyl oder $(C_{1-7})$-Alkylthio und $R^2$ Hydroxy oder di-$(C_1-C_7)$Alkyl-$N(CH_2)_n-NH-$ bedeuten, und n die in Anspruch 1 angegebene Bedeutung besitzt.

6. Verfahren gemäß Anspruch 2, dadurch gekennzeichnet, daß $R^1$ Hydroxy oder $(C_{1-7})$-Alkoxy und $R^2$ Hydroxy bedeuten.

7. Verfahren gemäß Anspruch 3, dadurch gekennzeichnet, daß $R^1$ $(C_{1-7})$-Alkylsulfinyl und $R^2$ Hydroxy bedeuten.

8. Verfahren gemäß einem der Ansprüche 1—4, dadurch gekennzeichnet, daß $R^2$ Hydroxy bedeutet.

9. Verfahren zur Herstellung von trans-3-(6-Isopropyl-4-oxo-4H-chinazolin-3-yl)-2-propensäure gemäß Anspruch 1, dadurch gekennzeichnet, daß man eine Verbindung der Formel Ic hydrolysiert, worin $R^1$ Isopropyl bedeutet.

10. Verfahren zur Herstellung von trans-3-(6-Methylthio-4-oxo-4H-chinazolin-3-yl)-2-propensäure gemäß Anspruch 1, dadurch gekennzeichnet, daß man eine Verbindung der Formel Ic hydrolysiert, worin $R^1$ Methylthio bedeutet.

11. Verfahren zur Herstellung von trans-3-(6-Isopropyl-4-oxo-4H-chinazolin-3-yl)-2-propensäure-2-diäthylaminoäthylamid gemäß Anspruch 1, dadurch gekennzeichnet, daß man eine Verbindung der Formel II, worin $R^{11}$ Isopropyl bedeutet, mit einem 3-Chloracrylsäure-Derivat der Formel III, worin $R^{21}$ 2-Diäthylamino bedeutet, umsetzt, oder ein aktives Derivat einer Carbonsäure der Formel Ig, worin $R^{11}$ Isopropyl bedeutet, mit 2-Diäthylaminoäthylamin behandelt.

12. Verfahren zur Herstellung von trans-3-(6-Isopropoxy-4-oxo-4H-chinazolin-3-yl)-2-propensäure gemäß Anspruch 1, dadurch gekennzeichnet, daß man eine Verbindung der Formel Ic hydrolysiert, worin $R^1$ Isopropoxy bedeutet.

13. Verfahren zur Herstellung von trans-3-(6-Hydroxy-4-oxo-4H-chinazolin-3-yl)-2-propensäure gemäß Anspruch 1, dadurch gekennzeichnet, daß man eine Verbindung der Formel Ic hydrolysiert, worin $R^1$ Hydroxy bedeutet.

14. Verfahren zur Herstellung von trans-3-(6-Methylsulfinyl-4-oxo-4H-chinazolin-3-yl)-2-propensäure gemäß Anspruch 1, dadurch gekennzeichnet, daß man eine Verbindung der Formel Ic, worin $R^1$ Methylsulfinyl bedeutet, hydrolysiert, oder eine Verbindung der Formel Ie, worin $R^{13}$ Methylthio bedeutet, oxydiert.

**Claims for the Contracting States: BE, CH, DE, FR, GB, IT, LI, LU, NL, SE**

1. Trans-quinazoline derivatives of general formula I

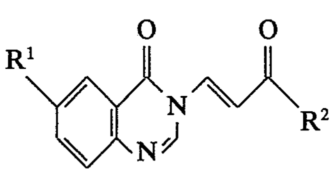

(I)

wherein $R^1$ signifies $(C_{1-7})$-alkyl, $(C_{3-7})$-cycloalkyl, $(C_{1-7})$-alkoxy, hydroxy, halogen, $(C_{1-7})$-alkylthio, $(C_{1-7})$-alkylsulphinyl, $(C_{1-7})$-alkylsulphonyl, di-$(C_1-C_7)$alkyl$-N(CH_2)_n-O-$, hydroxy-$(C_1-C_7)$-alkoxy, trifluoromethyl, nitro, amino, mono-$(C_{1-7})$-alkylamino or di-$(C_{1-7})$-alkylamino, $R^2$ signifies hydroxy, $(C_{1-7})$-alkoxy, di-$(C_1-C_7)$alkyl$-N(CH_2)_n-O-$ or di-$(C_1-C_7)$alkyl$-N(CH_2)_n-NH-$ and n signifies a whole number of 2—7,

and salts thereof with pharmaceutically acceptable bases when $R^2$ signifies hydroxy, or salts thereof with pharmaceutically acceptable acids when $R^1$ signifies amino, mono-$(C_{1-7})$-alkylamino, di-$(C_{1-7})$-alkylamino or di-$(C_1-C_7)$alkyl—$N(CH_2)_n$—O— and/or $R^2$ signifies di-$(C_1-C_7)$alkyl—$N(CH_2)_n$—O— or di-$(C_1-C_7)$alkyl—$N(CH_2)_n$—NH—.

2. Compounds according to claim 1 wherein $R^1$ signifies $(C_{1-7})$-alkyl, $(C_{3-7})$-cycloalkyl, $(C_{1-7})$-alkoxy, hydroxy, halogen, $(C_{1-7})$-alkylthio, di-$(C_1-C_7)$alkyl—$N(CH_2)_n$—O— or 2-hydroxy-ethoxy and n has the significance given in claim 1.

3. Compounds according to claim 1 wherein $R^1$ signifies $(C_{1-7})$-alkylsulphinyl or $(C_{1-7})$-alkylsulphonyl.

4. Compounds according to claim 2 wherein $R^1$ signifies $(C_{1-7})$-alkyl, $(C_{3-7})$-cycloalkyl, $(C_{1-7})$-alkoxy or $(C_{1-7})$-alkylthio.

5. Compounds according to claim 2 wherein $R^1$ signifies $(C_{1-7})$-alkyl or $(C_{1-7})$-alkylthio and $R^2$ signifies hydroxy or di-$(C_1-C_7)$alkyl—$N(CH_2)_n$—NH—, and n has the significance given in claim 1.

6. Compounds according to claim 2 wherein $R^1$ signifies hydroxy or $(C_{1-7})$-alkoxy and $R^2$ signifies hydroxy.

7. Compounds according to claim 3 wherein $R^1$ signifies $(C_{1-7})$-alkylsulphinyl und $R^2$ signifies hydroxy.

8. Compounds according to any one of claims 1—4 wherein $R^2$ signifies hydroxy.

9. Trans-3-(6-isopropyl-4-oxo-4H-quinazolin-3-yl)-2-propenoic acid.

10. Trans-3-(6-methylthio-4-oxo-4H-quinazolin-3-yl)-2-propenoic acid.

11. Trans-3-(6-isopropyl-4-oxo-4H-quinazolin-3-yl)-2-propenoic acid 2-(diethylamino)-ethylamide hydrochloride.

12. Trans-3-(6-isopropoxy-4-oxo-4H-quinazolin-3-yl)-2-propenoic acid.

13. Trans-3-(6-hydroxy-4-oxo-4H-quinazolin-3-yl)-2-propenoic acid.

14. Trans-3-(6-methylsulphinyl-4-oxo-4H-quinazolin-3-yl)-2-propenoic acid.

15. Trans-quinazolines of general formula VII

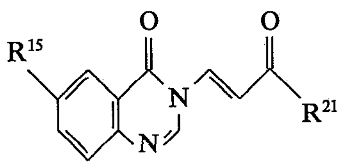

(VII)

wherein $R^{15}$ signifies a protected amino, mono-$(C_{1-7})$-alkylamino, hydroxy or hydroxy-$(C_{1-7})$-alkoxy group and $R^{21}$ signifies $(C_{1-7})$-alkoxy, di-$(C_1-C_7)$alkyl—$N(CH_2)_n$—O— or di-$(C_1-C_7)$-alkyl—$N(CH_2)_n$—NH—.

16. Compounds according to any one of claims 1—14 as pharmaceutically active substances.

17. Trans-3-(6-methylthio-4-oxo-4H-quinazolin-3-yl)-2-propenoic acid as a pharmaceutically active substance.

18. Compounds according to any one of claims 1—14 as anti-allergic active substances.

19. Trans-3-(6-methylthio-4-oxo-4H-quinazolin-3-yl)-2-propenoic acid as an anti-allergic active substance.

20. A process for the manufacture of trans-3-(4-oxo-4H-quinazolin-3-yl)-2-propenoic acid derivatives of general formula I according to claim 1 and of their pharmaceutically acceptable salts, characterized by

a) for the manufacture of compounds of general formula Ia

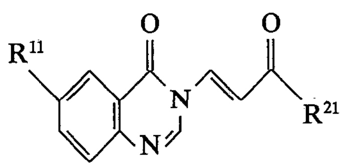

(Ia)

wherein $R^{11}$ signifies $(C_{1-7})$-alkyl, $(C_{3-7})$-cycloalkyl, $(C_{1-7})$-alkoxy, nitro, halogen, $(C_{1-7})$-alkylthio, $(C_{1-7})$-alkylsulphinyl, $(C_{1-7})$-alkylsulphonyl, trifluoromethyl, di-$(C_{1-7})$-alkylamino or di-$(C_1-C_7)$-alkyl—$N(CH_2)_n$—O—, $R^{21}$ signifies $(C_{1-7})$-alkoxy, di-$(C_1-C_7)$alkyl—$N(CH_2)_n$—O— or di-$(C_1-C_7)$-alkyl—$N(CH_2)_n$—NH and n signifies a whole number of 2—7, reacting a compound of general formula II

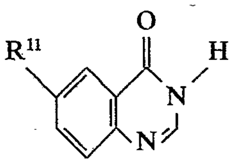

(II)

wherein $R^{11}$ has the above significance, with a 3-chloroacrylic acid derivative of general formula III

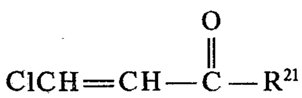

(III)

wherein $R^{21}$ has the above significance, or

b) for the manufacture of compounds of general formula I b

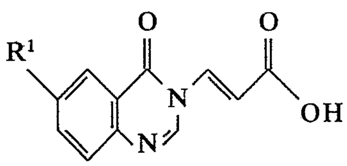

(I b)

wherein $R^1$ has the above significance, hydrolyzing a compound of general formula I c

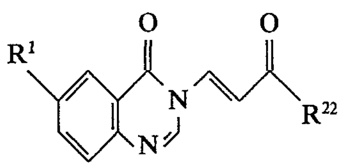

(I c)

wherein $R^{22}$ signifies $(C_{1-7})$-alkoxy and $R^1$ has the obove significance, or

c) for the manufacture of compounds of general formula I d

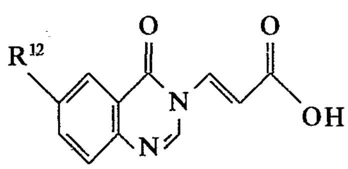

(I d)

wherein $R^{12}$ signifies $(C_{1-7})$-alkylsulphinyl or $(C_{1-7})$-alkylsulphonyl, oxidizing a compound of general formula I e

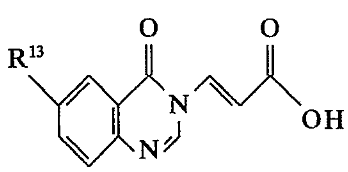

(I e)

wherein $R^{13}$ signifies $(C_{1-7})$-alkylthio, or

d) for the manufacture of compounds of general formula I f

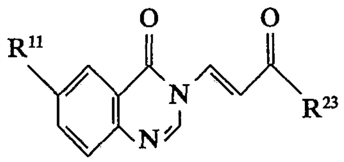

(If)

wherein $R^{23}$ signifies di-$(C_1{-}C_7)$alkyl–$N(CH_2)_n$–O– or di-$(C_1{-}C_7)$alkyl–$N(CH_2)_n$–NH– and $R^{11}$ and n have above significance, treating an active derivative of a carboxylic acid of general formula Ig

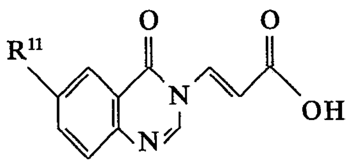

(Ig)

wherein $R^{11}$ has the above significance, with an alkanol of general formula IV

$$\text{di-}(C_1{-}C_7)\text{alkyl}{-}N(CH_2)_n{-}OH \qquad (IV)$$

or with an amine of general formula V

$$\text{di-}(C_1{-}C_7)\text{alkyl}{-}N(CH_2)_n{-}NH_2 \qquad (V)$$

wherein n in each case has the above significance, or

e) for the manufacture of compounds of general formula Ih

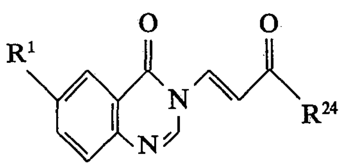

(Ih)

wherein $R^{24}$ di-$(C_1{-}C_7)$alkyl–$N(CH_2)_n$–O– and $R^1$ and n have the above significance, treating a carboxylic acid of general formula Ib above with a halide of general formula VI

$$\text{di-}(C_1{-}C_7)\text{alkyl}{-}N(CH_2)_n{-}X \qquad (VI)$$

wherein X signifies halogen and n has the above significance, or

f) for the manufacture of compounds of general formula Ii

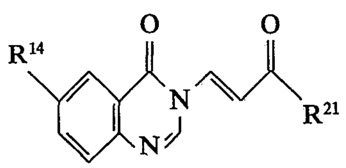

(Ii)

wherein $R^{14}$ signifies amino, mono-$(C_{1-7})$-alkylamino, hydroxy or hydroxy-$(C_{1-7})$-alkoxy and $R^{21}$ has the above significance, cleaving off the protecting group in a compound of general formula VII

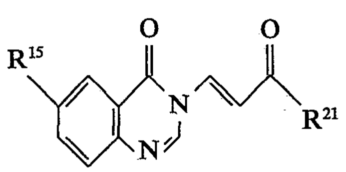

(VII)

wherein $R^{15}$ signifies a protected amino, mono-$(C_{1-7})$-alkylamino, hydroxy or hydroxy-$(C_{1-7})$-

alkoxy group and $R^{21}$ has the above significance, and

g) if desired, converting a compound of general formula I wherein $R^2$ signifies hydroxy into a pharmaceutically acceptable salt with a base, or converting a compound of general formula I wherein $R^1$ signifies amino, mono-$(C_{1-7})$-alkylamino, di-$(C_{1-7})$-alkylamino or di-$(C_1$—$C_7)$alkyl—$N(CH_2)_n$—O— and/or $R^2$ signifies di-$(C_1$—$C_7)$alkyl—$N(CH_2)_n$—O— or di-$(C_1$—$C_7)$alkyl—$N(CH_2)_n$—NH— and n has the above significance into a pharmaceutically acceptable acid addition salt.

21. A medicament containing a compound of general formula I given in claim 1 or a pharmaceutically acceptable salt thereof.

22. A medicament containing trans-3-(6-methylthio-4-oxo-4 H-quinazolin-3-yl)-2-propenoic acid or a pharmaceutically acceptable salt thereof.

23. An anti-allergic medicament containing a compound of general formula I given in claim 1 or a pharmaceutically acceptable salt thereof.

24. An anti-allergic medicament containing trans-3-(6-methylthio-4-oxo-4 H-quinazolin-3-yl)-2-propenoic acid or a pharmaceutically acceptable salt thereof.

**Claims for the Contracting State: AT**

1. A process for the manufacture of trans-quinazoline derivates of general formula I

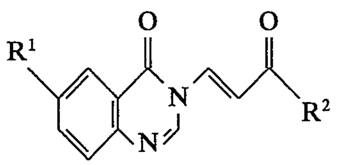

(I)

wherein $R^1$ signifies $(C_{1-7})$-alkyl, $(C_{3-7})$-cycloalkyl, $(C_{1-7})$-alkoxy, hydroxy, halogen, $(C_{1-7})$-alkylthio, $(C_{1-7})$-alkylsulphinyl, $(C_{1-7})$-alkylsulphonyl, di-$(C_1$—$C_7)$alkyl—$N(CH_2)_n$—O—, hydroxy-$(C_{1-7})$-alkoxy, trifluormethyl, nitro, amino, mono-$(C_{1-7})$-alkylamino or di-$(C_{1-7})$-alkylamino, $R^2$ signifies hydroxy, $(C_{1-7})$-alkoxy, di-$(C_1$—$C_7)$alkyl—$N(CH_2)_n$—O— or di-$(C_1$—$C_7)$alkyl—$N(CH_2)_n$—NH— and n signifies a whole number of 2—7,

and salts thereof with pharmaceutically acceptable bases when $R^2$ signifies hydroxy, or salts thereof with pharmaceutically acceptable acids when $R^1$ signifies amino, mono-$(C_{1-7})$-alkylamino, di-$(C_{1-7})$-alkylamino or di-$(C_1$—$C_7)$alkyl—$N(CH_2)_n$—O— and/or $R^2$ signifies di-$(C_1$—$C_7)$alkyl—$N(CH_2)_n$—O— or di-$(C_1$—$C_7)$alkyl—$N(CH_2)_n$—NH—, characterized by

a) for the manufacture of compounds of general formula I a

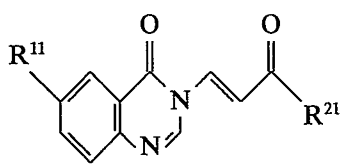

(I a)

wherein $R^{11}$ signifies $(C_{1-7})$-alkyl, $(C_{3-7})$-cycloalkyl, $(C_{1-7})$-alkoxy, nitro, halogen, $(C_{1-7})$-alkylthio, $(C_{1-7})$-alkylsulphinyl, $(C_{1-7})$-alkylsulphonyl, trifluormethyl, di-$(C_{1-7})$-alkylamino or di-$(C_1$—$C_7)$-alkyl-$N(CH_2)_n$—O—, $R^{21}$ signifies $(C_{1-7})$-alkoxy, di-$(C_1$—$C_7)$alkyl—$N(CH_2)_n$—O— or di-$(C_1$—$C_7)$-alkyl—$N(CH_2)_n$—NH and n signifies a whole number of 2—7,
reactin a compound of general formula II

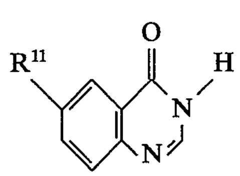

(II)

wherein $R^{11}$ has the above significance,
with a 3-chloroacrylic acid derivate of general formula III

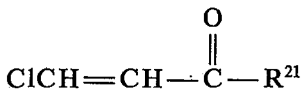

(III)

wherein $R^{21}$ has the above significance,
or

b) for the manufacture of compounds of general formula I b

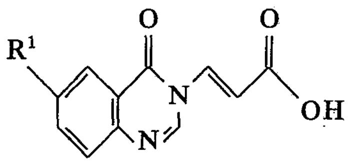

(Ib)

wherein $R^1$ has the above significance, hydrolyzing a compound of general formula I c

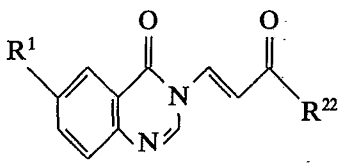

(Ic)

wherein $R^{22}$ signifies $(C_{1-7})$-alkoxy and $R^1$ has the above significance,
or

c) for the manufacture of compounds of general formula I d

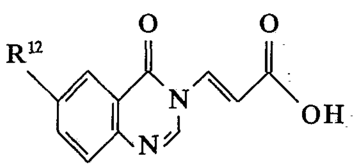

(Id)

wherein $R^{12}$ signifies $(C_{1-7})$-alkylsulphinyl or $(C_{1-7})$-alkylsulphonyl, oxidizing a compound of general formula I e

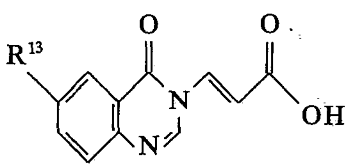

(Ie)

wherein $R^{13}$ signifies $(C_{1-7})$-alkylthio,
or

d) for the manufacture of compounds of general formula I f

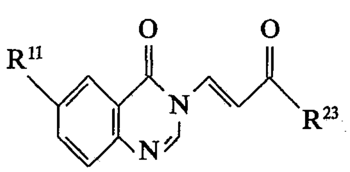

(If)

wherein $R^{23}$ signifies di-$(C_1{-}C_7)$alkyl$-N(CH_2)_n-O-$ or di-$(C_1{-}C_7)$alkyl$-N(CH_2)_n-NH-$ and $R^{11}$ and n have the above significance, treating an active derivative of a carboxylic acid of general formula I g

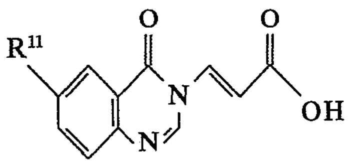

(Ig)

wherein $R^{11}$ has the above significance, with an alkanol of general formula IV

$$\text{di-}(C_1—C_7)\text{alkyl}—N(CH_2)_n—OH \qquad (IV)$$

or with an amine of general formula V

$$\text{di-}(C_1—C_7)\text{alkyl}—N(CH_2)_n—NH_2 \qquad (V)$$

wherein n in each case has the above significance,
or

e) for the manufacture of compounds of general formula I h

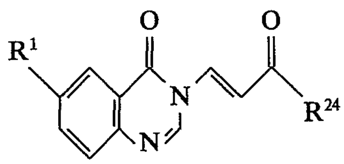

(Ih)

wherein $R^{24}$ signifies di-($C_1$—$C_7$)alkyl—N($CH_2$)$_n$—O— and $R^1$ and n have the above significance, treating a carboxylic acid of general formula I b above with a halide of general formula VI

$$\text{di-}(C_1—C_7)\text{alkyl}—N(CH_2)_n—X \qquad (VI)$$

wherein X signifies halogen and n has the above significance,
or

f) for the manufacture of compounds of general formula I i

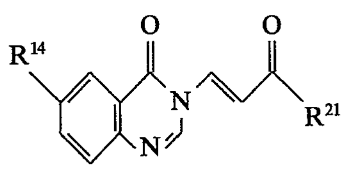

(Ii)

wherein $R^{14}$ signifies amino, mono-($C_{1-7}$)-alkylamino, hydroxy or hydroxy-($C_{1-7}$)-alkoxy and $R^{21}$ has the above significance, cleaving off the protecting group in a compound of general formula VII

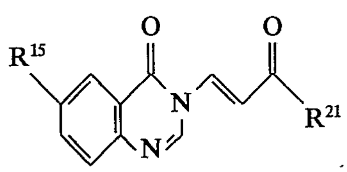

(VII)

wherein $R^{15}$ signifies a protected amino, mono-($C_{1-7}$)-alkylamino, hydroxy or hydroxy-($C_{1-7}$)-alkoxy group and $R^{21}$ has the above significance, and

g) if desired, converting a compound of general formula I wherein $R^2$ signifies hydroxy into a pharmaceutically acceptable salt with a base, or converting a compound of general formula I wherein $R^1$ signifies amino, mono-($C_{1-7}$)-alkylamino, di-($C_{1-7}$)-alkylamino or di-($C_1$—$C_7$)alkyl—N($CH_2$)$_n$—O— and/or $R^2$ signifies di-($C_1$—$C_7$)alkyl—N($CH_2$)$_n$—O— or di-($C_1$—$C_7$)alkyl—N($CH_2$)$_n$—NH— and n has the above significance into a pharmaceutically acceptable acid addition salt.

2. A process according to claim 1, characterized in that $R^1$ signifies ($C_{1-7}$)-alkyl, ($C_{3-7}$)-cycloalkyl, ($C_{1-7}$)-alkoxy, hydroxy, halogen, ($C_{1-7}$)-alkylthio, di-($C_1$—$C_7$)N($CH_2$)$_n$—O— or 2-hydroxyethoxy and n has the significance given in claim 1.

3. A process according to claim 1, characterized in that $R^1$ signifies ($C_{1-7}$)-alkylsulphinyl or ($C_{1-7}$)-

alkylsulphonyl.

4. A process according to claim 2, characterized in that $R^1$ signifies $(C_{1-7})$-alkyl, $(C_{1-7})$-cycloalkyl, $(C_{1-7})$-alkoxy or $(C_{1-7})$-alkylthio.

5. A process according to claim 2, characterized in that $R^1$ signifies $(C_{1-7})$-alkyl or $(C_{1-7})$-alkylthio and $R^2$ signifies hydroxy or di-$(C_1-C_7)$alkyl$-N(CH_2)_n-NH-$, and n has the significance given in claim 1.

6. A process according to claim 2, characterized in that $R^1$ signifies hydroxy or $(C_{1-7})$-alkoxy and $R^2$ signifies hydroxy.

7. A process according to claim 3, characterized in that $R^1$ signifies $(C_{1-7})$-alkylsulphinyl and $R^2$ signifies hydroxy.

8. A process according to any one of claim 1—4, characterized in that $R^2$ signifies hydroxy.

9. A process for the manufacture of trans-3-(6-isopropyl-4-oxo-4 H-quinazolin-3-yl)-2-propenoic acid in accordance with claim 1, characterized by hydrolyzing a compound of formula I c wherein $R^1$ signifies isopropyl.

10. A process for the manufacture of trans-3-(6-methylthio-4-oxo-4 H-quinazolin-3-yl)-2-propenoic acid in accordance with claim 1, characterized by hydrolyzing a compound of formula I c wherein $R^1$ signifies methylthio.

11. A process for the manufacture of trans-3-(6-isopropyl-4-oxo-4 H-quinazolin-3-yl)-2-propenoic acid 2-diethylaminoethylamide in accordance with claim 1, characterized by reacting a compound of formula II wherein $R^{11}$ signifies isopropyl with a 3-chloroacrylic acid derivative of formula III wherein $R^{21}$ signifies 2-diethylaminoethylamino, or treating an active derivative of a carboxylic acid of formula I g wherein $R^{11}$ signifies isopropyl with 2-diethylaminoethylamine.

12. A process for the manufacture of trans-3-(6-isopropoxy-4-oxo-4 H-quinazolin-3-yl)-2-propenoic acid in accordance with claim 1, characterized by hydrolyzing a compound of formula I c wherein $R^1$ signifies isopropoxy.

13. A process for the manufacture of trans-3-(6-hydroxy-4-oxo-4 H-quinazolin-3-yl)-2-propenoic acid in accordance with claim 1, characterized by hydrolyzing a compound of formula I c wherein $R^1$ signifies hydroxy.

14. A process for the manufacture of trans-3-(6-methylsulphinyl-4-oxo-4 H-quinazolin-3-yl)-2-propenoic acid in accordance with claim 1, characterized by hydrolyzing a compound of formula I c wherein $R^1$ signifies methylsulphinyl, or oxidizing a compound of formula I e wherein $R^{13}$ signifies methylthio.

**Revendications pour les Etats Contractants: BE, CH, DE, FR, GB, IT, LI, LU, NL, SE**

1. Dérivés de trans-quinazoline de formule générale I

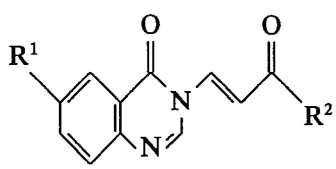

(I)

dans laquelle $R^1$ représente un groupe alkyle en $C_1-C_7$, cycloalkyle en $(C_3-C_7)$, alcoxy en $C_1-C_7$, hydroxy, halogène, alkylthio en $C_1-C_7$, alkylsulfinyle en $C_1-C_7$, alkylsulfonyle en $C_1-C_7$, di-(alkyle en $C_1-C_7$)$-N-(CH_2)_n-O-$, hydroxyalcoxy en $C_1-C_7$, trifluorométhyle, nitro, amino, monoalkylamino en $C_1-C_7$ ou di-(alkyle en $C_1-C_7$)-amino, $R^2$ représente un groupe hydroxy, alcoxy en $C_1-C_7$, di-(alkyle en $C_1-C_7$)$-N-(CH_2)_n-O-$ ou di-(alkyle en $C_1-C_7$)$-N-(CH_2)_n-NH-$ et n est un nombre entier allant de 2 à 7,

et, lorsque $R^2$ représente un groupe hydroxy, leurs sels avec des bases acceptables pour l'usage pharmaceutique, ou bien, lorsque $R^1$ représente un groupe amino, monoalkylamino en $C_1-C_7$, di-(alkyle en $C_1-C_7$)-amino ou di-(alkyle en $C_1-C_7$)$-N(CH_2)_n-O-$ et/ou $R^2$ représente un groupe di-(alkyle en $C_1-C_7$)$-N(CH_2)_n-O-$ ou di-(alkyle en $C_1-C_7$)$-N(CH_2)_n-NH-$, leurs sels avec des acides acceptables pour l'usage pharmaceutique.

2. Composés selon la revendication 1, dans lesquels $R^1$ représente un groupe alkyle en $C_1-C_7$, cycloalkyle en $C_1-C_7$, alcoxy en $C_1-C_7$, hydroxy, halogène, alkylthio en $C_1-C_7$, di-(alkyle en $C_1-C_7$)$-N(CH_2)_n-O-$ ou 2-hydroxyéthoxy, et n a la signification indiquée dans la revendication 1.

3. Composés selon la revendication 1, dans lesquels $R^1$ représente un groupe alkylsulfinyle en $C_1-C_7$ ou alkylsulfonyle en $C_1-C_7$.

4. Composés selon la revendication 2, dans lesquels $R^1$ représente un groupe alkyle en $C_1-C_7$, cycloalkyle en $C_3-C_7$, alcoxy en $C_1-C_7$ ou alkylthio en $C_1-C_7$.

5. Composés selon la revendication 2, dans lesquels $R^1$ représente un groupe alkyle en $C_1-C_7$ ou alkylthio en $C_1-C_7$, et $R^2$ représente un groupe hydroxy ou di-(alkyle en $C_1-C_7$)$-N(CH_2)_n-NH-$, et n a la

signification indiquée dans la revendication 1.

6. Composés selon la revendication 2, dans lesquels $R^1$ représente un groupe hydroxy ou alcoxy en $C_1$—$C_7$, et $R^2$ représente un groupe hydroxy.

7. Composés selon la revendication 3, dans lesquels $R^1$ représente un groupe alkylsulfinyle en $C_1$—$C_7$, et $R^2$ représente un groupe hydroxy.

8. Composés selon l'une quelconque des revendications 1 à 4, dans lesquels $R^2$ représente un groupe hydroxy.

9. L'acide trans-3-(6-isopropyl-4-oxo-4 H-quinazoline-3-yl)-2-propénoïque.

10. L'acide trans-3-(6-méthylthio-4-oxo-4 H-quinazoline-3-yl)-2-propénoïque.

11. Le chlorhydrate du 2-(diéthylamino)-éthylamide de l'acide trans-3-(6-isopropyl-4-oxo-4 H-quinazoline-3-yl)-2-propénoïque.

12. L'acide trans-3-(6-isopropoxy-4-oxo-4 H-quinazoline-3-yl)-2-propénoïque.

13. L'acide trans-3-(6-hydroxy-4-oxo-4 H-quinazoline-3-yl)-2-propénoïque.

14. L'acide trans-3-(6-méthylsulfinyl-4-oxo-4 H-quinazoline-3-yl)-2-propénoïque.

15. Trans-quinazolines de formule générale VII

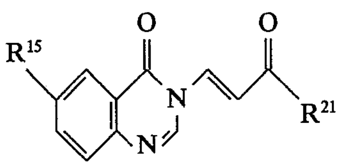

(VII)

dans laquelle $R^{15}$ représente un groupe amino, monoalkylamino en $C_1$—$C_7$, hydroxy ou hydroxyalcoxy en $C_1$—$C_7$ protégé, et $R^{21}$ représente un groupe alcoxy en $C_1$—$C_7$, di-(alkyle en $C_1$—$C_7$—$N(CH_2)_n$—O— ou di-(alkyle en $C_1$—$C_7$)—$N(CH_2)_n$—NH—.

16. Composés selon l'une quelconque des revendications 1 à 14, en tant que substances actives pharmaceutiques.

17. L'acide trans-3-(6-méthylthio-4-oxo-4 H-quinazoline-3-yl)-2-propénoïque en tant que substance active pharmaceutique.

18. Composé selon l'une quelconque des revendications 1 à 14 en tant que substances actives anti-allergiques.

19. L'acide trans-3-(6-méthylthio-4-oxo-4 H-quinazoline-3-yl)-2-propénoïque en tant que substance active anti-allergique.

20. Procédé de préparation des dérivés de l'acide trans-3-(4-oxo-4 H-quinazoline-3-yl)-2-propénoïque de formule générale I selon la revendication 1 et de leurs sels acceptables pour l'usage pharmaceutique, caractérisé en ce que

a) pour la préparation des composés de formule générale I a

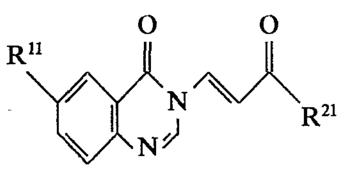

(I a)

dans laquelle $R^{11}$ représente un groupe alkyle en $C_1$—$C_7$, cycloalkyle en $C_3$—$C_7$, alcoxy en $C_1$—$C_7$, nitro, halogène, alkylthio en $C_1$—$C_7$, alkylsulfinyle en $C_1$—$C_7$, alkylsulfonyle en $C_1$—$C_7$, trifluorométhyle, di-(alkyle en $C_1$—$C_7$)-amino ou di-(alkyle en $C_1$—$C_7$)—$N(CH_2)_n$—O—, $R^{21}$ représente un groupe alcoxy en $C_1$—$C_7$, di-(alkyle en $C_1$—$C_7$)—$N(CH_2)_n$—O— ou di-(alkyle en $C_1$—$C_7$)—$N(CH_2)_n$—NH— et n est un nombre entier de 2 à 7,

on fait réagir un composé de formule générale II

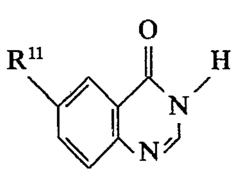

(II)

dans laquelle $R^{11}$ a la signification indiquée ci-dessus, avec un dérivé de l'acide 3-chloracrylique de formule générale II

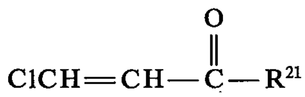

(III)

dans laquelle $R^{21}$ a la signification indiquée ci-dessus, ou bien

b) pour la préparation des composés de formule générale I b

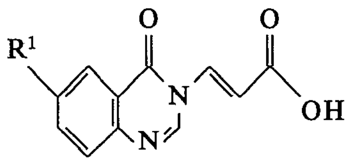

(Ib)

dans laquelle $R^1$ a la signification indiquée ci-dessus, on hydrolyse un composé de formule générale I c

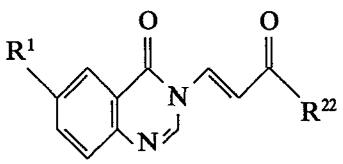

(Ic)

dans laquelle $R^{22}$ représente un groupe alcoxy en $C_1$—$C_7$, et $R^1$ a la signification indiquée ci-dessus, ou bien

c) pour la préparation des composés de formule générale I d

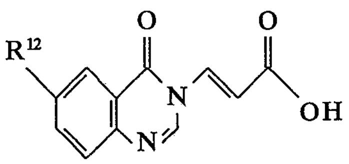

(Id)

dans laquelle $R^{12}$ représente un groupe alkylsulfinyle en $C_1$—$C_7$ ou alkylsulfonyle en $C_1$—$C_7$, on oxyde un composé de formule générale I e

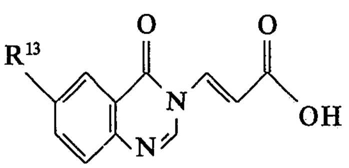

(Ie)

dans laquelle $R^{13}$ représente un groupe alkylthio en $C_1$—$C_7$, ou bien

d) pour la préparation des composés de formule générale I f

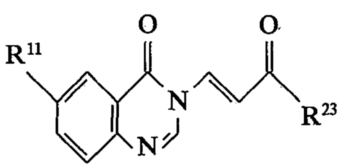

(If)

dans laquelle $R^{23}$ représente un groupe di-(alkyle en $C_1$—$C_7$)—$N(CH_2)_n$—O— ou di-(alkyle en $C_1$—$C_7$)—$N(CH_2)_n$—NH—, et $R^{11}$ et n ont les significations indiquées ci-dessus, on traite un dérivé actif d'un acide carboxylique de formule générale I g

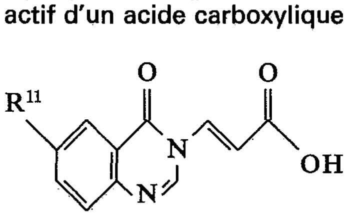

(Ig)

dans laquelle $R^{11}$ a la signification indiquée ci-dessus, par un alcanol de formule générale IV

di-(alkyle en $C_1$—$C_7$)—$N(CH_2)_n$—OH (IV)

ou par une amine de formule générale V

di-(alkyle en $C_1$—$C_7$)—$N(CH_2)_n$—$NH_2$ (V)

n ayant dans les deux cas la signification indiquée ci-dessus, ou bien

e) pour la préparation des composés de formule générale I h

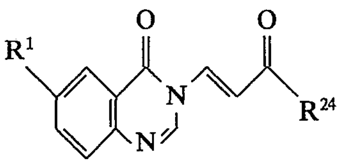

(Ih)

dans laquelle $R^{24}$ représente un groupe di-(alkyle en $C_1$—$C_7$)—$N(CH_2)_n$—O— et $R^1$ et n ont les significations indiquées ci-dessus, on traite un acide carboxylique de formule générale I b ci-dessus par un halogénure de formule générale VI

di-(alkyle en $C_1$—$C_7$)—$N(CH_2)_n$—X (VI)

dans laquelle X représente un halogène, et n a la signification indiquée ci-dessus, ou bien

f) pour la préparation des composés de formule générale I i

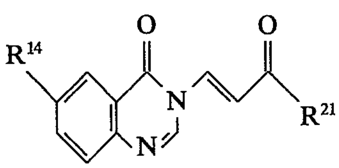

(I i)

dans laquelle $R^{14}$ représente un groupe amino, monoalkylamino en $C_1$—$C_7$, hydroxy ou hydroxyalcoxy en $C_1$—$C_7$, et $R^{21}$ a la signification indiquée ci-dessus, on élimine le groupe protecteur d'un composé de formule générale VII

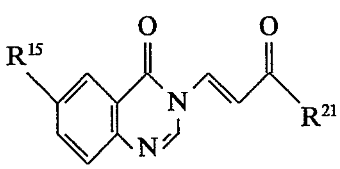

(VII)

dans laquelle $R^{15}$ représente un groupe amino, monoalkylamino en $C_1$—$C_7$, hydroxy ou hydroxyalcoxy en $C_1$—$C_7$ protégé, et $R^{21}$ a la signification indiquée ci-dessus, et

g) si on le désire, on convertit un composé de formule générale I dans laquelle $R^2$ représente un groupe hydroxy en un sel acceptable pour l'usage pharmaceutique à l'aide d'une base, ou bien on convertit un composé de formule générale I dans laquelle $R^1$ représente un groupe amino, monoalkylamino en $C_1$—$C_7$, di-(alkyle en $C_1$—$C_7$)-amino ou di-(alkyle en $C_1$—$C_7$)—$N(CH_2)_n$—O— et/ou $R^2$ représente un groupe di-(alkyle en $C_1$—$C_7$)—$N(CH_2)_n$—O— ou di-(alkyle en $C_1$—$C_7$)—$N(CH_2)_n$—NH— et n a la signification indiquée ci-dessus en un sel d'addition d'acide acceptable pour l'usage pharmaceutique.

21. Médicament contenant un composé de formule générale I indiquée dans la revendication I ou un de ses sels acceptables pour l'usage pharmaceutique.

22. Médicament contenant de l'acide trans-3-(6-méthylthio-4-oxo-4 H-quinazoline-3-yl)-2-propénoique ou un de ses sels acceptables pour l'usage pharmaceutique.

23. Médicament anti-allergique contenant un composé de formule générale I indiquée dans la revendication I ou de ses sels acceptables pour l'usage pharmaceutique.

24. Médicament anti-allergique contenant de l'acide trans-3-(6-méthylthio-4-oxo-4 H-quinazoline-3-yl)-2-propénoique ou un de ses sels acceptables pour l'usage pharmaceutique.

**Revendications pour l'Etat Contractant: AT**

1. Procédé pour la préparation de dérivés de trans-quinazoline de formule générale I

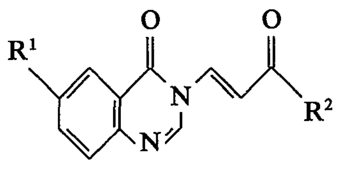

(I)

dans laquelle $R^1$ représente un groupe alkyle en $C_1-C_7$, cycloalkyle en ($C_3-C_7$), alcoxy en $C_1-C_7$, hydroxy, halogène, alkylthio en $C_1-C_7$, alkylsulfinyle en $C_1-C_7$, alkylsulfonyle en $C_1-C_7$, di-(alkyle en $C_1-C_7$)$-N-(CH_2)_n-O-$, hydroxyalcoxy en $C_1-C_7$, trifluorométhyle, nitro, amino, monoalkylamino en $C_1-C_7$ ou di-(alkyle en $C_1-C_7$)-amino, $R^2$ représente un groupe hydroxy, alcoxy en $C_1-C_7$, di-(alkyle en $C_1-C_7$)$-N-(CH_2)_n-O-$ ou di-(alkyle en $C_1-C_7$)$-N-(CH_2)_n-NH-$ et n est un nombre entier allant de 2 à 7,

et, lorsque $R^2$ représente un groupe hydroxy, de leurs sels avec des bases acceptables pour l'usage pharmaceutique ou bien, lorsque $R^1$ représente un groupe amino, monoalkylamino en $C_1-C_7$, di-(alkyle en $C_1-C_7$)-amino ou di-(alkyle en $C_1-C_7$)$-N(CH_2)_n-O-$ et/ou $R^2$ représente un groupe di-(alkyle en $C_1-C_7$)$-N(CH_2)_n-O-$ ou di-(alkyle en $C_1-C_7$)$-N(CH_2)_n-NH-$, de leurs sels avec des acides acceptables pour l'usage pharmaceutique, caractérisé en ce que

a) pour la préparation des composés de formule générale I a

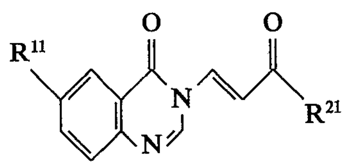

(I a)

dans laquelle $R^{11}$ représente un groupe alkyle en $C_1-C_7$, cycloalkyle en $C_3-C_7$, alcoxy en $C_1-C_7$, nitro, halogène, alkylthio en $C_1-C_7$, alkylsulfinyle en $C_1-C_7$, alkylsulfonyle en $C_1-C_7$, trifluorométhyle, di-(alkyle en $C_1-C_7$)-amino ou di-(alkyle en $C_1-C_7$)$-N(CH_2)_n-O-$, $R^{21}$ représente un groupe alcoxy en $C_1-C_7$, di-(alkyle en $C_1-C_7$)$-N(CH_2)_n-O-$ ou di-(alkyle en $C_1-C_7$)$-N(CH_2)_n-NH-$ et n est un nombre entier allant de 2 à 7,
on fait réagir un composé de formule générale II

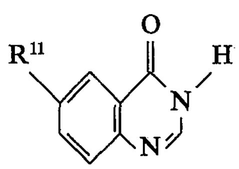

(II)

dans laquelle $R^{11}$ a la signification indiquée ci-dessus, avec un dérivé de l'acide 3-chloracrylique de formule générale II

$$ClCH{=}CH-\overset{\overset{O}{\|}}{C}-R^{21} \qquad (III)$$

dans laquelle $R^{21}$ a la signification indiquée ci-dessus, ou bien

b) pour la préparation des composés de formule générale I b

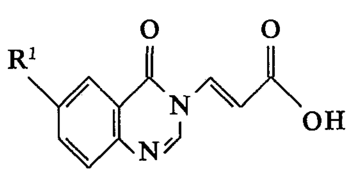

(Ib)

dans laquelle $R^1$ a la signification indiquée ci-dessus,

on hydrolyse un composé de formule générale I c

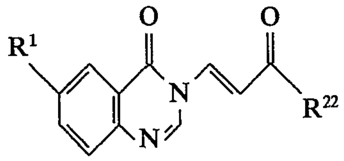

(I c)

dans laquelle $R^{22}$ représente un groupe alcoxy en $C_1$—$C_7$, et $R^1$ a la signification indiquée ci-dessus, ou bien

c) pour la préparation des composés de formule générale I d

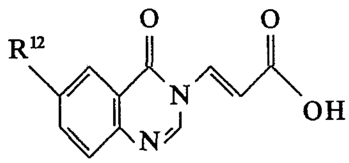

(I d)

dans laquelle $R^{12}$ représente un groupe alkylsulfinyle en $C_1$—$C_7$ ou alkylsulfonyle en $C_1$—$C_7$, on oxyde un composé de formule générale I e

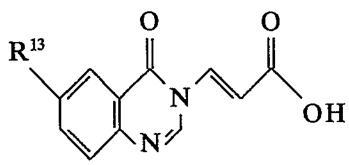

(I e)

dans laquelle $R^{13}$ représente un groupe alkylthio en $C_1$—$C_7$, ou bien

d) pour la préparation des composés de formule générale I f

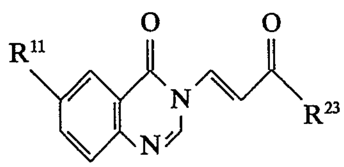

(I f)

dans laquelle $R^{23}$ représente un groupe di-(alkyle en $C_1$—$C_7$)—$N(CH_2)_n$—O— ou di-(alkyle en $C_1$—$C_7$)—$N(CH_2)_n$—NH—, et $R^{11}$ et n ont les significations indiquées ci-dessus, on traite un dérivé actif d'un acide carboxylique de formule générale I g

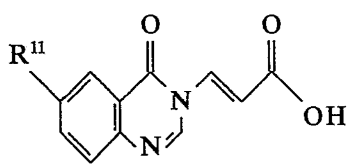

(I g)

dans laquelle $R^{11}$ a la signification indiquée ci-dessus, par un alcanol de formule générale IV

di-(alkyle en $C_1$—$C_7$)—$N(CH_2)_n$—OH (IV)

ou par une amine de formule générale V

di-(alkyle en $C_1$—$C_7$)—$N(CH_2)_n$—$NH_2$ (V)

n ayant dans les deux cas la signification indiquée ci-dessus, ou bien

e) pour la préparation des composés de formule générale I h

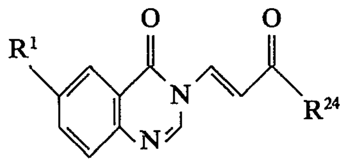

(Ih)

dans laquelle $R^{24}$ représente un groupe di-(alkyle en $C_1$—$C_7$)—$N(CH_2)_n$—O— et $R^1$ et n ont les significations indiquées ci-dessus, on traite un acide carboxylique de formule générale I b ci-dessus par un halogénure de formule générale VI

di-(alkyle en $C_1$—$C_7$)—$N(CH_2)_n$—X (VI)

dans laquelle X représente un halogène, et n a la signification indiquée ci-dessus, ou bien

f) pour la préparation des composés de formule générale I i

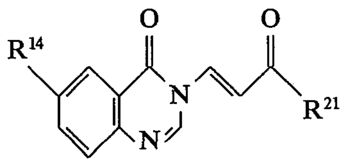

(I i)

dans laquelle $R^{14}$ représente un groupe amino, monoalkylamino en $C_1$—$C_7$, hydroxy ou hydroxyalcoxy en $C_1$—$C_7$, et $R^{21}$ a la signification indiquée ci-dessus, on élimine le groupe protecteur d'un composé de formule générale VII

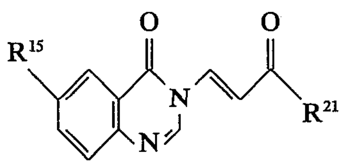

(VII)

dans laquelle $R^{15}$ représente un groupe amino, monoalkylamino en $C_1$—$C_7$, hydroxy ou hydroxyalcoxy en $C_1$—$C_7$ protégé, et $R^{21}$ a la signification indiquée ci-dessus, et

g) si on le désire, on convertit un composé de formule générale I dans laquelle $R^2$ représente un groupe hydroxy on un sel acceptable pour l'usage pharmaceutique à l'aide d'une base, ou bien on convertit un composé de formule générale I dans laquelle $R^1$ représente un groupe amino, monoalkylamino en $C_1$—$C_7$, di-(alkyle en $C_1$—$C_7$)-amino ou di-(alkyle en $C_1$—$C_7$)—$N(CH_2)_n$—O— et/ou $R^2$ représente un groupe di-(alkyle en $C_1$—$C_7$)—$N(CH_2)_n$—O— ou di-(alkyle en $C_1$—$C_7$)—$N(CH_2)_n$—NH— et n a la signification indiquée ci-dessus, en un sel d'addition d'acide acceptable pour l'usage pharmaceutique.

2. Procédé selon la revendication 1, caractérisé en ce que $R^1$ représente un groupe alkyle en $C_1$—$C_7$, cycloalkyle en $C_3$—$C_7$, alcoxy en $C_1$—$C_7$, hydroxy, halogène, alkylthio en $C_1$—$C_7$, di(alkyle en $C_1$—$C_7$)—$N(CH_2)_n$—O— ou 2-hydroxyéthoxy, et n a la signification indiquée dans la revendication 1.

3. Procédé selon la revendication 1, caractérisé en ce que $R^1$ représente un groupe alkylsulfinyle en $C_1$—$C_7$ ou alkylsulfonyle en $C_1$—$C_7$.

4. Procédé selon la revendication 2, caractérisé en ce que $R^1$ représente un groupe alkyle en $C_1$—$C_7$, cycloalkyle en $C_3$—$C_7$, alcoxy en $C_1$—$C_7$ ou alkylthio en $C_1$—$C_7$.

5. Procédé selon la revendication 2, caractérisé en ce que $R^1$ représente un groupe alkyle en $C_1$—$C_7$ ou alkylthio en $C_1$—$C_7$, et $R^2$ représente un groupe hydroxy ou di-(alkyle en $C_1$—$C_7$)—$N(CH_2)_n$—NH—, et n a la signification indiquée dans la revendication 1.

6. Procédé selon la revendication 2, caractérisé en ce que $R^1$ représente un groupe hydroxy ou alcoxy en $C_1$—$C_7$, et $R^2$ un groupe hydroxy.

7. Procédé selon la revendication 3, caractérisé en ce que $R^1$ représente un groupe alkylsulfinyle en $C_1$—$C_7$, et $R^2$ un groupe hydroxy.

8. Procédé selon l'une quelconque des revendications 1 à 4, caractérisé en ce que $R^2$ représente un groupe hydroxy.

9. Procédé de préparation de l'acide trans-3-(6-isopropyl-4-oxo-4 H-quinazoline-3-yl)-2-propénoïque selon la revendication 1, caractérisé en ce que l'on hydrolyse un composé de formule I c dans laquelle $R^1$ représente un groupe isopropyle.

10. Procédé de préparation de l'acide trans-3-(6-méthylthio-4-oxo-4 H-quinazoline-3-yl)-2-propénoïque selon la revendication 1, caractérisé en ce que l'on hydrolyse un composé de formule I c dans

laquelle $R^1$ représente un groupe méthylthio.

11. Procédé de préparation du 2-diéthylaminoéthylamide de l'acide trans-3-(6-isopropyl-4-oxo-4 H-quinazoline-3-yl)-2-propénoïque selon la revendication 1, caractérisé en ce que l'on fait réagir un composé de formule II dans laquelle $R^{11}$ représente un groupe isopropyle avec un dérivé de l'acide 3-chloracrylique de formule III dans laquelle $R^{21}$ représente un groupe 2-diéthylaminoéthylamino, ou bien on traite un dérivé actif d'un acide carboxylique de formule I g dans laquelle $R^{11}$ représente le groupe isopropyle, par la 2-diéthylaminoéthylamine.

12. Procédé de préparation de l'acide trans-3-(6-isopropoxy-4-oxo-4 H-quinazoline-3-yl)-2-propénoïque selon la revendication 1, caractérisé en ce que l'on hydrolyse un composé de formule I c dans laquelle $R^1$ représente un groupe isopropoxy.

13. Procédé de préparation de l'acide trans-3-(6-hydroxy-4-oxo-4 H-quinazoline-3-yl)-2-propénoïque selon la revendication 1, caractérisé en ce que l'on hydrolyse un composé de formule I c dans laquelle $R^1$ représente un groupe hydroxy.

14. Procédé de préparation de l'acide trans-3-(6-méthylsulfinyl-4-oxo-4 H-quinazoline-3-yl)-2-propénoïque selon la revendication 1, caractérisé en ce que l'on hydrolyse un composé de formule I c dans laquelle $R^1$ représente un groupe méthylsulfinyle, ou bien on oxyde un composé de formule I c dans laquelle $R^{13}$ représente un groupe méthylthio.